# EUROPEAN PATENT APPLICATION

(11) **EP 4 790 720 A1**
(43) Date of publication of application: **12.08.2026**
(21) Application number: 26155642.7
(22) Date of filing: 02.02.2026
(51) Int. Cl.: G16H 40/20, G16H 40/60, H04W 4/80, H04W 84/12

(54) **WIRELESS CONNECTION OF MEDICAL DEVICES WITH OPERATING ROOM WIRELESS NETWORK**

(30) Priority: 05.02.2025 US 202563754346 P
(71) Applicant: STRYKER CORPORATION, Portage, MI 49002-9711 (US)
(72) Inventor: Sharma, Sheetal, 143105 Punjab (IN); Jain, Arpeet, 122004 Gurgaon (IN); Adamo, Andrew, Willow Park, 76087 (US); Gupta, Deepak, Haryana (IN); Kumar, Avinash, 827003 Jarkhand (IN); Mehta, Sudhanshu, 143532 Punjab (IN); Stanaland, Courtney, Flower Mound, 75028 (US); Musko, John, 75038 Irving (US); Obi, Aghogho, 33327 Weston (US); Obireddygari, Suryanarayana Reddy, 515775 Anantapur (IN); Hernandez, Cameron, 34476 Ocala, FL (US); Wildgen, Michael R., 49002 Portage, MI (US)
(74) Representative: Schott, Jakob Valentin

(57) **Abstract**

Systems and methods for connecting a medical device to a wireless network are provided. The operating room may include an operating room communication device and/or a hub device configured to transmit wireless network information. The medical device may connect with the wireless network based on the wireless network information.

## Description

### TECHNICAL FIELD

The present disclosure relates generally to establishing wireless connection between devices in an operating room.

### BACKGROUND

Typically, a hospital may include many operating rooms, where medical operations may be carried out using assigned medical devices. In instances where more and more medical devices are configured for wireless communication, these medical devices may transmit and/or receive information related to the assigned medical operation and/or operation room. There remains a need in the art to ensure that the medical devices transmit and/or receive information corresponding to the assigned medical operation and/or operation room.

### SUMMARY

According to a first aspect, a system for connecting a medical device to a wireless network is provided, the system including: an operating room communication device configured to: receive a broadcast signal; and transmit wireless network information for connecting wirelessly with the wireless network in response to receiving the broadcast signal; and a medical device configured to: transmit the broadcast signal to the operating room communication device; receive wireless network information from the operating room communication device; and transmit the wireless network information to initiate connection with the wireless network.

According to a second aspect, a method for operating a communication is provided, the communication system including a medical device and an operating room communication device, the method including steps of: transmitting, with the medical device, a broadcast signal; receiving, with the operating room communication device, the broadcast signal; transmitting, with the operating room communication device, wireless network information for connecting wirelessly with the wireless network in response to receiving the broadcast signal; receiving, with the medical device, the wireless network information from the operating room communication device; and transmitting, with the medical device, the wireless network information to initiate connection with the wireless network.

According to a third aspect, a system for connecting a medical device to a wireless network is provided, the system comprising: a medical device configured to perform a function; a control device in communication with the medical device, the control device being configured to transmit a control signal, wherein the medical device is configured to perform the function in response to receiving the control signal transmitted by the control device; and a hub device being configured to transmit wireless network information for connecting wirelessly with the wireless network to the control device; wherein the control device is configured to transmit the wireless network information to the medical device; and wherein the medical device is configured to transmit the wireless network information to initiate connection with the wireless network.

According to a fourth aspect, a method of operating a communication system is provided, the communication system including a medical device, a hub device, and a control device, the method including steps of: transmitting, with the control device, a control signal; receiving, with the medical device, the control signal transmitted by the control device; performing, with the medical device, a function based on the control signal transmitted by the control device; transmitting, with the hub device, wireless network information for connecting wirelessly with the wireless network; receiving, with the control device, the wireless network information from the hub device; transmitting, with the control device, the wireless network information to the medical device; and transmitting, with the medical device, the wireless network information to initiate connection with the wireless network.

According to a fifth aspect, a system for connecting a medical device to a wireless network is provided, the system comprising: a medical device configured to perform a function; a control device being configured to: transmit wireless network information to the medical device, wherein the medical device is configured to transmit the wireless network information to initiate connection with the wireless network; and transmit a control signal, wherein, in response to the control device being connected with the medical device, the medical device is configured to perform the function based on the control signal transmitted by the control device.

According to a sixth aspect, a method of operating a communication system is provided, the communication system including a medical device and a control device, the method including steps of: transmitting, with the control device, wireless network information to the medical device in response to the medical device being connected with the control device; transmitting, with the medical device, the wireless network information to initiate connection with the wireless network; transmitting, with the control device, a control signal; receiving, with the medical device, the control signal transmitted by the control device; and performing, with the medical device, a function in response to the medical device being connected with the control device based on the control signal transmitted by the control device.

According to a seventh aspect, a system for connecting a medical device with a wireless network is provided, the system including: a configuration application configured to transmit wireless network information; an operating room communication device configured to: receive a broadcast signal; receive the wireless network information from the configuration application; and transmit the wireless network information for connecting wirelessly with the wireless network in response to receiving the broadcast signal; and a medical device configured to: transmit the broadcast signal to the operating room communication device; receive wireless network information from the operating room communication device; and transmit the wireless network information to initiate connection with the wireless network.

According to an eighth aspect, a system for connecting a medical device with a wireless network is provided, the system including: an operating room communication device configured to: receive a broadcast signal; and transmit wireless network information using an ultrawide band signal for connecting wirelessly with the wireless network in response to receiving the broadcast signal; and a medical device configured to: transmit the broadcast signal to the operating room communication device using an ultrawide band signal; receive wireless network information from the operating room communication device; determine a time-of-flight of the ultrawide band signal transmitted by the operating room communication device; determine a distance between the operating room communication device and the medical device based on the time-of-flight of the ultrawide band signal; determine that the distance between the operating room communication device and the medical device is within a predetermined distance threshold; and transmit the wireless network information to initiate connection with the wireless network in response to determining that the distance between the operating room communication device and the medical device is within the predetermined distance threshold.

According to a ninth aspect, a system for connecting a medical device to a wireless network is provided, the system comprising: a medical device; a control device in communication with the medical device, the control device being configured to transmit a control signal, wherein the medical device is configured to receive the control signal and perform a function based on the control signal transmitted by the control device; and a hub device configured to electrically connect with the control device via a 1-Wire communication interface, the hub device being configured to transmit wireless network information for connecting wirelessly with the wireless network to the control device via the 1-Wire communication device; wherein the control device is configured to transmit the wireless network information to the medical device; and wherein the medical device is configured to transmit the wireless network information to initiate connection with the wireless network.

According to a tenth aspect, a system for connecting a medical device to a wireless network is provided, the system comprising: a medical device configured to perform a function; a control device being configured to: electrically connect with the medical device via a 1-Wire communication interface; transmit wireless network information to the medical device via the 1-Wire communication interface, wherein the medical device is configured to transmit the wireless network information to initiate connection with the wireless network; and transmit a control signal, wherein, in response to the control device being connected with the medical device, the medical device is configured to receive the control signal and perform the function based on the control signal.

Any of the above aspects may be combined in-part or in-whole with any of the implementations below.

In one implementation, the system further includes a wireless access point in communication with the wireless network. In one implementation, the medical device is configured to transmit the wireless network information to the wireless access point to initiate connection with the wireless network. In one implementation, the wireless network information includes pairing information for initiating connection with the wireless access point.

In one implementation, the system further includes a hub device configured to connect with the medical device and transmit a control signal to the medical device after the hub device has connected with the medical device. In one implementation, the medical device is configured to transmit the wireless network information to the hub device to initiate connection with the wireless network. In one implementation, the wireless network information includes pairing information for initiating connection with the hub device.

In one implementation, the system further includes a configuration application configured to transmit the wireless network information to the operating room communication device, and wherein the operating room communication device is configured to receive the wireless network information from the configuration application prior to transmitting the wireless network information.

In one implementation, the system further includes a power source coupled to the operating room communication device, wherein the operating room communication device is configured to operate in: a sleep mode where the power source provides a first amount of power to the operating room communication device; and an active mode where the power source provides a second amount of power to the operating room communication device, where the first amount of power is less than the second amount of power.

In one implementation, in response to receiving the broadcast signal, the operating room communication device is configured to transition from the sleep mode to the active mode, and the operating room communication device is configured to transmit the wireless network information to the medical device in response to the operating room communication device transitioning from the sleep mode to the active mode. In one implementation, the medical device is configured to transmit a sleep signal to the operating room communication device in response to the medical device being connected with the wireless network, and the operating room communication device is configured to transition from the active mode to the sleep mode in response to receiving the sleep signal.

In one implementation, the medical device is configured to transmit the broadcast signal using an ultrawide band signal, and the operating room communication device is configured to transmit wireless network information using an ultrawide band signal. In one implementation, the medical device includes a controller and a transmitter, wherein the controller is configured to: determine a time-of-flight of the ultrawide band signal transmitted by the operating room communication device; and control the transmitter to transmit the wireless network information to initiate connection with the wireless network based on the time-of-flight of the ultrawide band signal. In one implementation, the medical device includes a controller and a transmitter, wherein the controller is configured to: determine a time-of-flight of the ultrawide band signal transmitted by the operating room communication device; and terminate connection of the medical device and the wireless network based on the time-of-flight of the ultrawide band signal.

In one implementation, the controller is configured to: determine a distance between the operating room communication device and the medical device based on the time-of-flight of the ultrawide band signal; determine that the distance between the operating room communication device and the medical device is within a predetermined distance threshold; and control the transmitter to transmit the wireless network information to initiate connection with the wireless network in response to determining that the distance between the operating room communication device and the medical device is within the predetermined distance threshold. In one implementation, the controller is configured to: determine a distance between the operating room communication device and the medical device based on the time-of-flight of the ultrawide band signal; determine that the distance between the operating room communication device and the medical device is greater than a predetermined distance threshold; and terminate connection of the medical device and the wireless network in response to determining that the distance between the operating room communication device and the medical device is greater than the predetermined distance threshold.

In one implementation, the wireless network is further defined as a first wireless network, the operating room communication device is further defined as a first operating room communication device, and further includes a second operating room communication device configured to: receive the broadcast signal; and transmit wireless network information for connecting wirelessly with the second wireless network in response to receiving the broadcast signal.

In one implementation, the medical device includes a controller and a transmitter, wherein the controller is configured to: control the transmitter to transmit the wireless network information for connecting wirelessly with the first wireless network to initiate connection with the first wireless network; and control the transmitter to transmit the wireless network information for connecting wirelessly with the second wireless network to initiate connection with the second wireless network.

In one implementation, the medical device including a user interface is configured to receive a user input; wherein the controller is configured to control the transmitter to transmit the wireless network information for connecting wirelessly with the first wireless network or the wireless network information for connecting wirelessly with the second wireless network based on the user input.

In one implementation, the medical device is configured to terminate connection of the medical device and the wireless network. In one implementation, the medical device is further defined as a first medical device, and further includes a second medical device connected with the wireless network, and wherein the first medical device is configured to communicate with the second medical device in response to the first medical device being connected with the wireless network.

In one implementation, the communication system further includes a wireless access point in communication with the wireless network. In one implementation, the step of transmitting, with the medical device, the wireless network information to initiate connection with the wireless network further includes a step of transmitting, with the medical device, the wireless network information to the wireless access point to initiate connection with the wireless access point. In one implementation, the communication system further includes a hub device, and the method further comprises steps of: establishing connection between the hub device and the medical device; and transmitting, with the hub device, a control signal to the medical device.

In one implementation, the step of transmitting, with the medical device, the wireless network information to initiate connection with the wireless network further includes a step of transmitting, with the medical device, the wireless network information to the hub device to initiate connection with the wireless network. In one implementation, the step of transmitting, with the medical device, the broadcast signal further includes a step of transmitting, with the medical device, the broadcast signal using an ultrawide band signal, and wherein the step of transmitting, with the operating room communication device, the wireless network information further includes a step of transmitting, with the operating room communication device, the wireless network information with an ultrawide band signal.

In one implementation, the medical device includes a controller and a transmitter, and the method further comprises steps of: determining, with the controller, a time-of-flight of the ultrawide band signal transmitted by the operating room communication device; and controlling, with the controller, the transmitter to transmit the wireless network information to initiate connection with the wireless network based on the time-of-flight of the ultrawide band signal.

In one implementation, the method further comprises a step of: determining, with the controller, a distance between the operating room communication device and the medical device based on the time-of-flight of the ultrawide band signal; determining, with the controller, that the distance between the operating room communication device and the medical device is within a predetermined distance threshold; and controlling, with the controller, the transmitter to transmit the wireless network information to initiate connection with the wireless network in response to determining that the distance between the operating room communication device and the medical device is within the predetermined distance threshold.

In one implementation, the method further comprises a step of terminating, with the medical device, connection of the medical device with the wireless network. In one implementation, the step of transmitting, with the medical device, the broadcast signal further includes a step of transmitting, with the medical device, the broadcast signal using an ultrawide band signal, and wherein the step of transmitting, with the operating room communication device, the wireless network information further includes a step of transmitting, with the operating room communication device, the wireless network information with an ultrawide band signal.

In one implementation, the medical device includes a controller and a transmitter, and the method further comprises steps of: determining, with the controller, a time-of-flight of the ultrawide band signal transmitted by the operating room communication device; determining, with the controller, a distance between the operating room communication device and the medical device based on the time-of-flight of the ultrawide band signal; determining, with the controller, that the distance between the operating room communication device and the medical device is greater than a predetermined distance threshold; and terminating, with the medical device, connection of the medical device with the wireless network in response to determining that the distance between the operating room communication device and the medical device is greater than a predetermined distance threshold.

In one implementation, the system further includes a wireless access point in communication with the wireless network. In one implementation, the medical device is configured to transmit the wireless network information to the wireless access point to initiate connection with the wireless network. In one implementation, the medical device is configured to transmit the wireless network information to the hub device to initiate connection with the wireless network. In one implementation, the hub device is configured to transmit a control signal, and wherein, in response to the medical device being connected with the wireless network, the medical device is configured to receive the control signal transmitted by the hub device and perform the function based on the control signal transmitted by the hub device.

In one implementation, the medical device is configured to transmit operational information to the hub device in response to the medical device being connected with the wireless network. In one implementation, the hub device includes a display device, the display device being configured to display the operational information. In one implementation, the control device is configured to transmit a broadcast signal to the hub device using a near field communication signal, and wherein the hub device is configured to transmit the wireless network information in response to receiving the broadcast signal.

In one implementation, the control device and the hub device are configured to electrically connect. In one implementation, the hub device is coupled to a docking station sized to receive the control device, and the control device and the hub device are configured to electrically connect in response to the control device being received by the docking station. In one implementation, the hub device and the control device are configured to electrically connect via a 1-Wire communication interface, and wherein the hub device is configured to transmit wireless network information to the control device via the 1-Wire communication interface.

In one implementation, the control device is configured to connect wirelessly with the medical device such that the control signal is transmitted via the wireless connection. In one implementation, the control device is configured to electrically connect with the medical device such that the control signal is transmitted via the electrical connection. In one implementation, the control device and the medical device are configured to electrically connect via a 1-Wire communication interface, and wherein the control device is configured to transmit wireless network information to the medical device via the 1-Wire communication interface.

In one implementation, the communication system further includes a wireless access point in communication with the wireless network, and the method further comprises a step of transmitting, with the medical device, the wireless network information to the wireless access point to initiate connection with the wireless network. In one implementation, the method further comprises a step of transmitting, with the medical device, the wireless network information to the hub device to initiate connection with the wireless network.

In one implementation, the method further comprises a step of transmitting, with the control device, a broadcast signal to the hub device. In one implementation, the step of transmitting, with the hub device, wireless network information for connecting wirelessly with the wireless network comprises a step of transmitting, with the hub device, wireless network information for connecting wirelessly with the wireless network in response to receiving the broadcast signal. In one implementation, the method further comprises a step of electrically connecting the control device and the hub device.

In one implementation, the method further comprises steps of: transmitting, with the hub device, a control signal; and receiving, with the medical device, the control signal transmitted by the hub device; performing, with the medical device, a function based on the control signal transmitted by the hub device in response to the medical device being connected with the wireless network. In one implementation, the method further comprises a step of transmitting, with the medical device, at least one of health information of the medical device and patient information in response to the medical device being connected with the wireless network.

In one implementation, the system further includes a wireless access point in communication with the wireless network. In one implementation, the medical device is configured to transmit the wireless network information to the wireless access point to initiate connection with the wireless network. In one implementation, the system further comprises a hub device in communication with the wireless network. In one implementation, the medical device is configured to transmit the wireless network information to the hub device to initiate connection with the wireless network.

In one implementation, the hub device is configured to transmit a control signal, and wherein, in response to the medical device being connected with the wireless network, the medical device is configured to receive the control signal transmitted by the hub device and perform the function based on the control signal transmitted by the hub device. In one implementation, the medical device is configured to transmit operational information to the hub device in response to the medical device being connected with the wireless network.

In one implementation, the hub device includes a display device, the display device being configured to display the operational information. In one implementation, the control device is configured to transmit wireless network information to the medical device using a near field communication signal. In one implementation, the control device and the medical device are configured to electrically connect. In one implementation, the medical device is coupled to a docking station sized to receive the control device, and wherein the control device and the medical device are configured to electrically connect in response to the control device being received by the docking station. In one implementation, the control device is configured to connect wirelessly with the medical device such that the control signal is transmitted via the wireless connection.

In one implementation, the communication system further includes a wireless access point in communication with the wireless network, and the method further comprises a step of transmitting, with the medical device, the wireless network information to the wireless access point to initiate connection with the wireless network.

In one implementation, the communication system further includes a hub device in communication with the wireless network, and the method further comprises steps of: transmitting, with the medical device, the wireless network information to the hub device to initiate connection with the wireless network; transmitting, with the hub device, a control signal; receiving, with the medical device, the control signal transmitted by the hub device; and performing, with the medical device, a function based on the control signal transmitted by the hub device in response to the medical device being connected with the wireless network.

In one implementation, the method further comprises a step of transmitting, with the medical device, at least one of health information of the medical device and patient information in response to the medical device being connected with the wireless network. In one implementation, the method further comprises a step of transmitting, with the control device, wireless network information to the medical device using a near field communication signal. In one implementation, the method further comprises a step of electrically connecting the control device and the hub device.

### BRIEF DESCRIPTION OF THE DRAWINGS

Advantages of the present invention will be readily appreciated as the same becomes better understood by reference to the following detailed description when considered in connection with the accompanying drawings.
FIG. 1 is a perspective view of a first implementation of a communication system including a medical device and an operating room communication device.
FIG. 2A is a schematic view of the first implementation shown in FIG. 1.
FIG. 2B is a schematic view of the first implementation shown in FIG. 1, further including a configuration application.
FIG. 3A illustrates a flowchart of a method of establishing communication between the wireless network and the medical device of the first implementation shown in FIG. 1.
FIG. 3B illustrates a flowchart of a method of establishing communication between the wireless network and the medical device of the first implementation shown in FIG. 1, wherein the wireless network information is transmitted by a configuration application.
FIG. 4 is a schematic view of the first implementation of the communication system shown in FIG. 1, where more than one operating room communication device is positioned in an operating room.
FIG. 5 is a schematic view of the first implementation of the communication system shown in FIG. 1, where more than one operating room communication device is positioned in an operating room and where the medical device and the wireless network have established communication.
FIG. 6A is diagrammatic view of an in-range and out-range of an operating room communication device.
FIG. 6B is diagrammatic view of an in-range and out-range of a first operating room communication device and a second operating room communication device.
FIG. 7 is a perspective view of an instance of a second implementation of a communication system including a medical device, a hub device, and a control device.
FIG. 8 is a schematic view of the instance of the second implementation of the communication system shown in FIG. 6.
FIG. 9 illustrates a flowchart of a method of establishing communication between the wireless network and the medical device.
FIGS. 10A and 10B illustrate various examples of the control device.
FIGS. 11A and 11B illustrate a touchscreen display of the control device during the establishing of communication between the wireless network and the medical device.
FIG. 12 illustrates the touchscreen display of the control device providing a notification after communication has been established between the medical device and the hub device.
FIG. 13 illustrates a touchscreen display of the hub device providing a notification after communication has been established between the medical device and the hub device.
FIG. 14 illustrates the control device providing a control signal to the medical device.
FIG. 15 is a perspective view of an instance of a second implementation of a communication system including a medical device, a hub device, and a control device.
FIG. 16 is a schematic view of the instance of the second implementation shown in FIG. 15.
FIG. 17 illustrates a flowchart of a method of establishing communication between the wireless network and the medical device of the instance of the second implementation shown in FIG. 15.

### DETAILED DESCRIPTION

### I. OVERVIEW OF THE COMMUNICATION SYSTEM

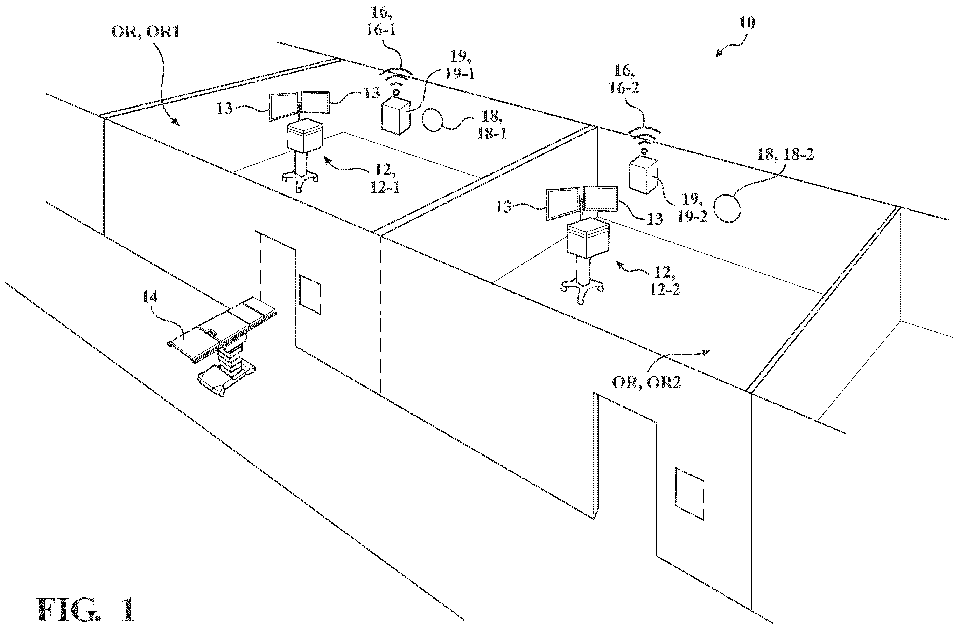

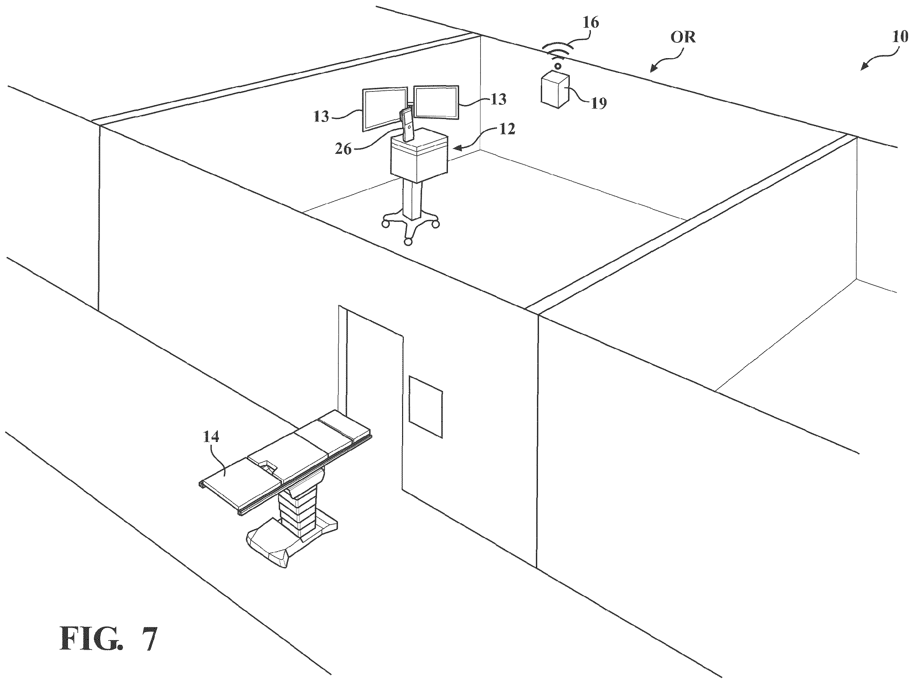

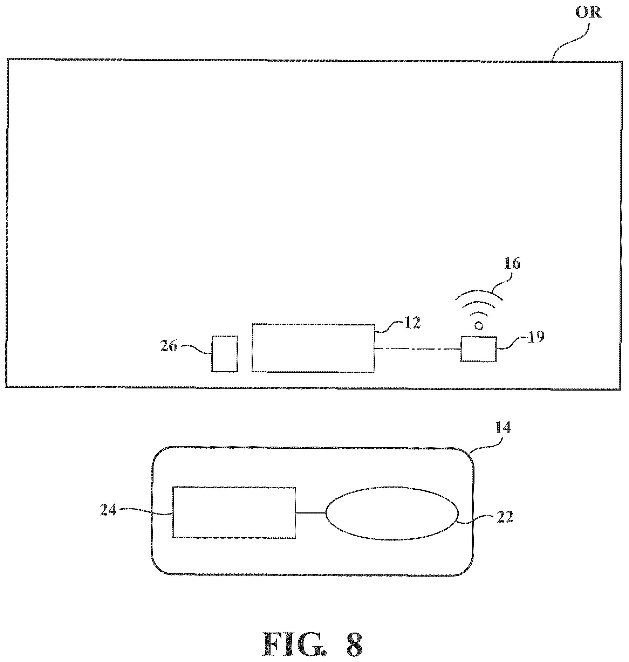

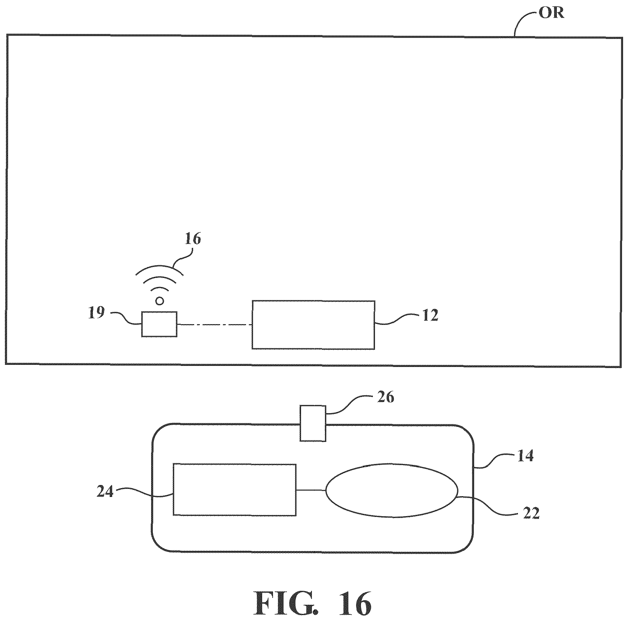

A communication system for connecting a medical device to a wireless network is described herein. A first implementation of a communication system 10 is shown in FIGS. 1-6B. A second implementation of the communication system 10 is shown in FIGS. 7-17. For each implementation, the communication system 10 includes a medical device 14 and a wireless network 16, wherein communication is established between the medical device 14 and the wireless network 16.

Communication may be established between the wireless network 16 and the medical device 14 such that the medical device 14 may upload and download information related to a medical procedure.

In some instances, once communication is established between the medical device 14 and the wireless network, the medical device 14 may upload (transmit) and download (receive) information via the wireless network 16. For instance, the medical device 14 may upload (transmit) and download (receive) information from a remote computing device via the wireless network 16. In some instances, the remote computing device may be within an operation room in which the medical device 14 is located. In some instances, the remote computing device may be outside the operation room. In some instances, the remote computing device may be one or more cloud servers. As an example, the medical device 14 may receive a software update from the remote computing device via the wireless network 16. As another example, the medical device 14 may receive operational parameters for performing a medical procedure from the remote computing device via the wireless network 16. As yet another example, the medical device 14 may transmit operational data related to the medical procedure (e.g. a profile of a surgeon and/or preferences of the surgeon) and/or a status of the medical device 14 (e.g. health parameters of the medical device 14, such as battery health, component failure, service requirements of the medical device 14, and/or in-use status of the medical device 14) to the remote computing device via the wireless network 16. As will be explained in greater detail below, the medical device 14 may be any medical device 14 configured to perform a function related to a medical operation. For example, the medical device 14 may include a patient support apparatus configured to support a patient. In such instances, the status of the medical device 14 may include an orientation of the patient support apparatus, a state of the patient support apparatus (e.g. an articulation of the patient support apparatus), a configuration of the patient support apparatus (e.g. what modules are attached to the patient support apparatus and at what location), an amount of mass supported by the patient support apparatus, etc. As another example, the medical device 14 may include a fluid management system configured to supply fluids. In such instances, the status of the medical device 14 may include fluid volume captured during a surgical procedure, vacuum pressure, etc.

In some instances, once communication is established between the medical device 14 and the wireless network, the medical device 14 may transmit and receive information from other devices connected with the wireless network 16. For example, once communication is established between the medical device 14 and the wireless network 16, the medical device 14 may communicate with other medical devices 14 connected with the wireless network 16 (to be described in greater detail below).

The medical device 14 may be connected with a wireless network 16 to assign the medical device 14 to a corresponding operating room OR. As shown in FIG. 1, an operating room OR may correspond to a wireless network 16. As shown, a first wireless network 16-1 corresponds to a first operating room OR1 and a second wireless network 16-2 corresponds to a second operating room OR2. A medical device 14 assigned to an operating room OR communicates via the corresponding wireless network 16. For example, in the instance of FIG. 1, the medical device 14 may be connected with the first wireless network 16-1 such that the medical device 14 is assigned to the first operating room OR1. As follows, the medical device 14 communicates via the first wireless network 16-1. For instance, the medical device 14 may upload and download information from a remote computing device via the first wireless network 16-1 and the medical device 14 may transmit and receive information from other devices connected with the first wireless network 16-1. As another example, in the instance of FIG. 1, the medical device 14 may be connected with the second wireless network 16-2 such that the medical device 14 is assigned to the second operating room OR2. As follows, the medical device 14 communicates via the second wireless network 16-2. For instance, the medical device 14 may upload and download information from a remote computing device via the second wireless network 16-2 and the medical device 14 may transmit and receive information from other devices connected with the second wireless network 16-2.

The wireless network 16 may be any suitable wireless network. For example, the wireless network 16 may be a cellular/mobile network, a wireless personal area network (PAN), a wireless local area network (WLAN), and/or a wireless ad hoc network. The wireless network 16 may be configured to communicate using any suitable communication protocol, such as any one or more of, ultra-wideband, Bluetooth, near field communication (NFC), infrared (LiFi), Zigbee, WiFi, cellular, and/or WiMAX communication signals.

In some instances, the communication system 10 may include a wireless access point 19 configured to serve as a gateway for connecting the medical device 14 to the wireless network 16. In such instances, the medical device 14 may connect with the wireless network 16 by connecting with the wireless access point 19. The wireless access point 19 may include any device that may be in communication with the wireless network 16. The wireless access point 19 may be in wired or wireless communication with the wireless network 16. For example, in instances where the wireless network 16 is a WiFi network, the wireless access point 19 may include a wireless router in wired communication with the wireless network 16 via an Ethernet cable. As another example, in instances where the wireless network 16 is a cellular network, the wireless access point 19 may include a cellular router/mobile hotspot in wireless communication with the wireless network 16.

The communication system 10 may include any suitable number of wireless access points 19. For example, in some instances, the number of wireless access points 19 may correspond with the number of wireless networks 16. In the instance shown in FIG. 1, a first wireless access point 19-1 corresponds to the first wireless network 16-1, and second wireless access point 19-2 corresponds to the second wireless network 16-2. In such instances, the first wireless access point 19-1 may serve as a gateway for connecting the medical device 14 and the first wireless network 16-1 and the second wireless access point 19-2 may serve as a gateway for connecting the medical device 14 and the second wireless network 16-2.

The medical device 14 and the wireless access point 19 may include any suitable transmitters, receivers, and/or transceivers for connecting with one another. The medical device 14 and the wireless access point 19 may be configured to connect with one another using any suitable wireless pairing protocol, including but not limited to Bluetooth, Wi-Fi, NFC, Zigbee, Ultra-Wideband (UWB), or other short-range or long-range protocols. Accordingly, a type of the transmitters, receivers, and/or transceivers of the medical device 14 and the wireless access point 19 may correspond to the wireless protocol. For instance, in an instance where the medical device 14 and the wireless access point 19 are configured to connect using Bluetooth pairing technology, the medical device 14 and the wireless access point 19 may include a Bluetooth transceiver.

The medical device 14 may be any medical device configured to perform a function related to a medical operation. For example, the function may be a medical operation, a part of the medical operation, to prepare for a medical operation, and/or to assist a medical operation. In the first and second implementations described herein, the medical device 14 is shown as a patient support apparatus. The patient support apparatus is further described as the transport system in PCT Application Serial No. PCT/US2024/036637 entitled "Foot Plate For Patient Support Apparatus Of Surgical System For Use With Medical Imaging", the disclosure of which is hereby incorporated by reference in its entirety.

The medical device 14 may include any device and/or system for supporting a patient. For example, the medical device 14 may include the patient transport apparatus in U.S. Patent No. 11,389,348 entitled "Patient Transport Apparatus Having Powered Drive System Utilizing Dual Mode User Input Control", the patient transport apparatus in U.S. Patent No. 11,679,45 entitled "Patient Transport Apparatus User Interface", the patient transport apparatus described in U.S. Patent No. 11,806,296 entitled "Patient Transport Apparatus With Controlled Auxiliary Wheel Speed", the patient transport apparatus described in U.S. Patent No. 11,833,085 entitled "Patient Transport Apparatus With Steer Lock Assembly", the patient handling device described in U.S. Patent No. 9,27,183 entitled "Patient Handling Device", and/or the patient support apparatus described in U.S. Patent No. 12,29,694 entitled "Patient Support Apparatus With Hold Mode", the disclosures of which are hereby incorporated by reference in their entirety.

The medical device 14 may include a surgical console and a surgical instrument, where the surgical console may control an actuator of a surgical instrument. For example, the medical device 14 may include the surgical console and the instrument described in U.S. Patent No. 11,759,271 entitled "System And Method For Indicating Mapping Of Console-Based Surgical Systems", the control console and the ultrasonic handpiece described in U.S. Patent No. 10,16,209 entitled "System and Method for Driving an Ultrasonic Handpiece as a Function of the Mechanical Impedance of the Handpiece", the control console and the electrodes described in U.S. Patent No. 11,497,543 entitled "Control Console And Accessories For RF Nerve Ablation And Methods Of Operating The Same", the control system and the pump described in U.S. Patent No. 9,474,848 entitled "Fluid Management System", the and/or the control console described in U.S. Patent No. 10,449,570 entitled "System and Method for Driving an Ultrasonic Handpiece with a Linear Amplifier," the disclosures of which are hereby incorporated by reference in their entirety.

The medical device 14 may include a surgical instrument configured to perform an operation on a patient. For example, the medical device 14 may include the surgical handpiece assembly of U.S. Patent No. 11,317,927 entitled "Measurement Module For Measuring Depth Of Bore Holes And Related Accessories" and/or the surgical tool system of U.S. Patent No. 12,29,439 entitled "Surgical Tool System With Interchangeable Battery And Control Modules", the disclosures of which are hereby incorporated by reference in their entirety.

The medical device 14 may include a system for charging a battery of a surgical instrument. For example, the medical device 14 may include the system of U.S. Patent No. 12,34,315 entitled "System And Method For Wirelessly Charging A Medical Device Battery", the disclosure of which is hereby incorporated by reference in its entirety.

The medical device 14 may include any device including a processor and/or a memory. For example, the medical device 14 may include any controller including a processor and/or memory. Similarly, the medical device 14 may include any suitable computing device, such as a computer, a tablet device, a smartphone device, and/or a head-mounted display device.

The medical device 14 may include any user interface suitable for receiving a user input. For example, the medical device 14 may include one or more touchscreen displays configured to receive a user input from an operator of the communication system 10. As another example, the user interface may include a trigger, a button, a directional pad, and/or a user-actuatable switch. As yet another example, the user interface may include any computing device, such as a computer, a tablet device, a smartphone device, and/or a head-mounted display device for receiving a user input. Additionally, the medical device 14 may include an output device configured to provide an output to an operator of the communication system 10. For example, the medical device 14 may include a display for providing visual information to the operator, such as a touchscreen display and/or a head-mounted display. As another example, the medical device 14 may include light emitting diodes (LEDs) configured to emit light, a speaker for providing an audible notification, and/or a vibratory device configured to provide tactile feedback.

The communication system 10 may include any suitable number of medical devices 14. For example, more than one medical device 14 may be used in a medical procedure. In such instances, the more than one medical devices 14 may be connected with the wireless network 16. Additionally, once the medical devices 14 are connected with the wireless network 16, the medical devices 14 may communicate with one another to receive/transmit information. Referring to FIG. 1, the medical device 14 may communicate with other medical devices 14 connected with the first wireless network 16-1 in response to the medical device 14 being connected with the first wireless network 16-1 and the medical device 14 may communicate with other medical devices 14 connected with the second wireless network 16-2 in response to the medical device 14 being connected with the second wireless network 16-2. For example, the medical device 14 may receive operational data related to the medical procedure (e.g. a profile of a surgeon and/or preferences of the surgeon) and/or a status of other medical devices 14 (e.g. health parameters of other medical devices 14, such as battery health, component failure, service requirements of other medical devices 14, and/or in-use status of other medical devices 14) from the other medical devices 14. An output device of the medical device 14 may provide a notification to an operator of the communication system 10 based on the operational data related to the medical procedure and/or status received from the other medical devices 14.

The communication system 10 may include a hub device 12 configured to be connected with the medical device 14. The medical device 14 and the hub device 12 may include any suitable transmitters, receivers, and/or transceivers for connecting with one another. The medical device 14 and the hub device 12 may be configured to connect with one another using any suitable wireless pairing protocol, including but not limited to Bluetooth, Wi-Fi, NFC, Zigbee, Ultra-Wideband (UWB), or other short-range or long-range protocols. Accordingly, a type of the transmitters, receivers, and/or transceivers of the medical device 14 and the hub device 12 may correspond to the wireless protocol.

The hub device 12 may include a processor and/or a memory. For example, the medical device 14 may include any controller including a processor and/or memory. Similarly, the medical device 14 may include any suitable computing device, such as a computer, a tablet device, a smartphone device, and/or a head-mounted display device.

The hub device 12 may include a user interface configured to receive an input from an operator of the communication system 10. For example, in the first and second implementations described herein, the hub device 12 may include one or more touchscreen displays 13 configured to receive a user input from a user. In other implementations, the user interface may include a trigger, a button, a directional pad, and/or a user-actuatable switch. Additionally, the user interface may include any computing device, such as a computer, a tablet device, a smartphone device, and/or a head-mounted display device for receiving a user input. Additionally, the hub device 12 may include an output device configured to provide an output to an operator of the communication system 10. For example, a display, such as a touchscreen display 13 of the hub device 12 may provide visual information to the operator. As another example, the hub device 12 may include light emitting diodes (LEDs) configured to emit light, a speaker for providing an audible notification, and/or a vibratory device configured to provide tactile feedback.

Additionally, the hub device 12 may be the hub device described in PCT Application Serial No. PCT/US2024/011608 entitled "System For Controlling Multiple Surgical Consoles With Multiple Control Devices", the disclosure of which is hereby incorporated by reference in its entirety.

The hub device 12 may be configured to communicate with the medical device 14 in response to the medical device 14 being connected with the hub device 12.

For example, once the medical device 14 is connected with the hub device 12, the hub device 12 may provide a control signal to the medical device 14 via the wireless network 16. For instance, an operator may provide a user input to a touchscreen display 13 of the hub device 12 and the hub device 12 may provide a corresponding control signal to the medical device 14 via the wireless network 16. Accordingly, the medical device may receive the control signal provided by the hub device 12 and perform a function based on the control signal transmitted by the hub device 12. In an example instance where the medical device 14 includes a patient transport apparatus, an actuator of the patient transport apparatus may be actuated based on the control signal transmitted by the hub device 12. For instance, a lift system of the patient transport apparatus may be actuated to lift a patient support surface of the patient transport apparatus based on the control signal transmitted by the hub device 12. As another example, in instances where the medical device 14 includes a surgical console, the surgical console may provide a control signal to a surgical instrument based on the control signal transmitted by the hub device 12. As another example, in instances where the medical device 14 includes a surgical instrument, a controller of the surgical instrument may provide a control signal to an actuator of the surgical instrument based on the control signal transmitted by the hub device 12. As another example, in instances where the medical device 14 includes a computing device, the computing device may receive the control signal transmitted by the hub device as an input and perform a corresponding function. For instance, a computing device may run a program in response to receiving the control signal transmitted by the hub device 12.

As another example, once the medical device 14 is connected with the hub device 12, the hub device 12 may transmit to and/or receive information from the medical device 14. For example, the medical device 14 may receive operational data related to the medical procedure (e.g. a profile of a surgeon and/or preferences of the surgeon) and/or a status of the hub device 12 (e.g. health parameters of the hub device 12, such as battery health, component failure, service requirements of the hub device 12, and/or in-use status of the hub device 12) from the hub device 12. An output device of the medical device 14 may provide a notification to an operator of the communication system 10 based on the operational data related to the medical procedure and/or status received from the hub device 12. As another example, the hub device 12 may receive operational data related to the medical procedure (e.g. a profile of a surgeon and/or preferences of the surgeon) and/or a status of the medical device 14 (e.g. health parameters of the medical device 14, such as battery health, component failure, service requirements of the medical device 14, and/or an in-use status of the medical device 14) from the medical device 14. An output device of the hub device 12, such as a touchscreen display 13, may provide a notification to an operator of the communication system 10 based on the operational data and/or status received from the medical device 14. Additionally, in some instances, the hub device 12 may provide a software update to the medical device 14.

The hub device 12 may be connected with the wireless network 16. The hub device 12 may upload and download information from a remote computing device via the wireless network 16. For example, the hub device 12 may receive a software update from a cloud server via the wireless network 16. As another example, the hub device 12 may transmit operational data related to a medical procedure to a cloud server via the wireless network 16. As yet another example, the hub device 12 may receive operational parameters for performing a medical procedure from a cloud server via the wireless network 16.

The hub device 12 may be in wired or wireless communication with the wireless network 16. For example, the hub device 12 may be wirelessly connected with wireless network 16 via the wireless access point 19. In such instances, the hub device 12 and the wireless access point 19 may include any suitable transmitters, receivers, and/or transceivers for connecting with one another. The hub device 12 and the wireless access point 19 may be configured to connect with one another using any suitable wireless pairing protocol, including but not limited to Bluetooth, Wi-Fi, NFC, Zigbee, Ultra-Wideband (UWB), or other short-range or long-range protocols. Accordingly, a type of the transmitters, receivers, and/or transceivers of the hub device 12 and the wireless access point 19 may correspond to the wireless protocol. As another example, the hub device 12 may be connected with the wireless network 16 via an Ethernet cable.

In some instances, as an alternative to the wireless access point 19, or in addition to the wireless access point 19, the hub device 12 may be configured to serve as a gateway for connecting the medical device 14 to the wireless network 16. In such instances, the medical device 14 may connect with the wireless network 16 by connecting with the hub device 12. For example, in instances where the wireless network 16 is a WiFi network, the hub device 12 may include a wireless router in wired communication with the wireless network 16 via an Ethernet cable. As another example, in instances where the wireless network 16 is a cellular network, the hub device 12 may include a cellular router/mobile hotspot in wireless communication with the wireless network 16.

The communication system 10 may include any suitable number of hub devices 12. For example, in some instances, the number of hub devices 12 may correspond with the number of operating rooms OR. In the instance shown in FIG. 1, a first hub device 12-1 is positioned in and corresponds to the first operating room OR1, and a second hub device 12-2 is positioned in and corresponds to the second operating room OR2. In such instances, the first hub device 12-1 may be connected with the first wireless network 16-1 and the second hub device 12-2 may be connected with the second wireless network 16-2. Additionally, the first hub device 12-1 may communicate with the medical device 14 in response to the medical device 14 being connected with the first hub device 12-1 and the second hub device 12-2 may communicate with the medical device 14 in response to the medical device 14 being connected with the second hub device 12-2.

### II. COMMUNICATION SYSTEM (FIRST IMPLEMENTATION)

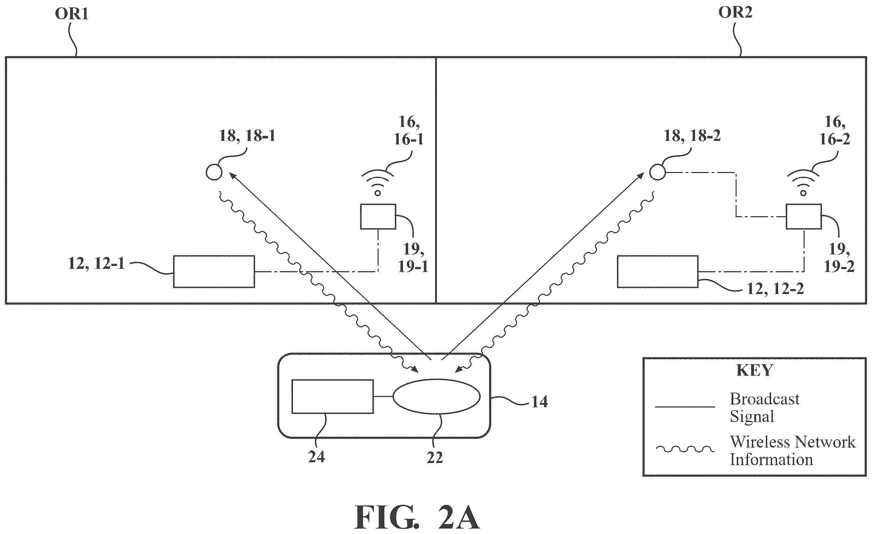

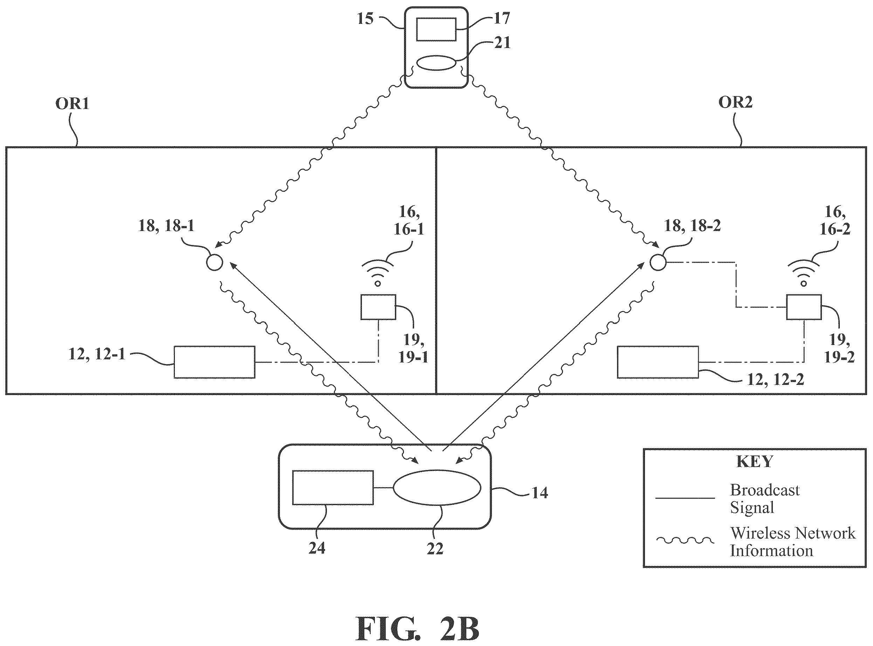

The first implementation of the communication system 10 is shown in FIGS. 1-6B. In the first implementation of the communication system 10, the medical device 14 is connected with a wireless network 16 corresponding to an operating room OR. For example, referring to FIGS. 1, 2A, and 2B, the medical device 14 may be connected with the first wireless network 16-1 corresponding to the first operating room OR1 or with the second wireless network 16-2 corresponding to the second operating room OR2. As previously stated, the medical device 14 may be connected with a wireless network 16 to assign the medical device 14 to the corresponding operating room OR. In the instance of FIG. 1, the medical device 14 may be connected with either the first wireless network 16-1 or the second wireless network 16-2 to assign the medical device 14 to either the first operating room OR1 or the second operating room OR2. As follows, the medical device 14 may communicate via either the first wireless network 16-1 or via the second wireless network 16-2.

In the first implementation of the communication system 10, the communication system 10 includes an operating room communication device 18. The operating room communication device 18 facilitates connection between the medical device 14 and the wireless network 16. More particularly, the operating room communication device 18 and the medical device 14 are configured to communicate and the medical device 14 may connect with the wireless network 16 based on the communication. For example, the operating room communication device 18 may transmit wireless network information to the medical device 14 and the medical device 14 may connect with the wireless network 16 using the wireless network information.

The wireless network information may be used for pairing the medical device 14 and the wireless network 16. The wireless network information encompasses the data used for two devices to establish a secure and reliable connection across various communication protocols. This information typically includes network identifiers, such as SSIDs for Wi-Fi or device names for Bluetooth, along with authentication credentials like passwords, PIN codes, or cryptographic keys. It may also involve protocol-specific parameters, including frequency channels, encryption standards, and supported profiles, as well as device capability details and service discovery information. Depending on the wireless pairing protocol, including but not limited to Bluetooth, Wi-Fi, NFC, Zigbee, Ultra-Wideband (UWB), or other short-range or long-range protocols, additional security tokens or certificates may be exchanged to ensure encrypted and authenticated communication. Collectively, these elements enable devices to recognize each other, negotiate compatibility, and establish a secure link for data exchange.

FIG. 2A illustrates the communication between the operating room communication device 18 and the medical device 14. As shown, the medical device 14 transmits a broadcast signal to the operating room communication device 18 and the operating room communication device 18 transmits wireless network information for connecting wirelessly with the wireless network 16 to the medical device 14 in response to receiving the broadcast signal.

The medical device 14 and the operating room communication device 18 may include any suitable transmitters, receivers, and/or transceivers for communicating with one another. For example, the medical device 14 and the operating room communication device 18 may be configured to transmit the broadcast signal and the wireless network information using any suitable wireless protocol and a type of the transmitters, receivers, and/or transceivers of the medical device 14 and the operating room communication device 18 may correspond to the wireless protocol. For instance, the medical device 14 and the operating room communication device 18 may be configured to transmit the broadcast signal and the wireless network information using any suitable communication protocol, such as any one or more of radio, ultra-wideband (UWB), Bluetooth, near field communication (NFC), infrared, Zigbee, WiFi, cellular, and/or WiMAX communication signals. In an example instance, the medical device 14 may transmit the broadcast signal using a UWB signal and the operating room communication device 18 may transmit the wireless network information using a UWB signal. Additionally, a range of wireless communication between the medical device 14 and the operating room communication device 18 may correspond to a range provided by the wireless protocol used by the medical device 14 and the operating room communication device 18. For example, in instances where the medical device 14 and the operating room communication device 18 communicate using NFC communication signals, the range of communication between the medical device 14 and the operating room communication device 18 may correspond to a range of NFC communication signals. Similarly, in instances where the medical device 14 and the operating room communication device 18 communicate using WiFi communication signals, the range of communication between the medical device 14 and the operating room communication device 18 may correspond to a range of WiFi communication signals.

Additionally, the transmitters, receivers, and/or transceivers of the medical device 14 and the operating room communication device 18 may be an active or passive device. In some instances, the transmitters, receivers, and/or transceivers of the medical device 14 and the operating room communication device 18 may be coupled to a power source (e.g. a battery, wall outlet, etc.). In other instances, the transmitters, receivers, and/or transceivers of the medical device 14 and the operating room communication device 18 may be energized by radio signal (e.g. an RF signal, a UWB signal, etc.).

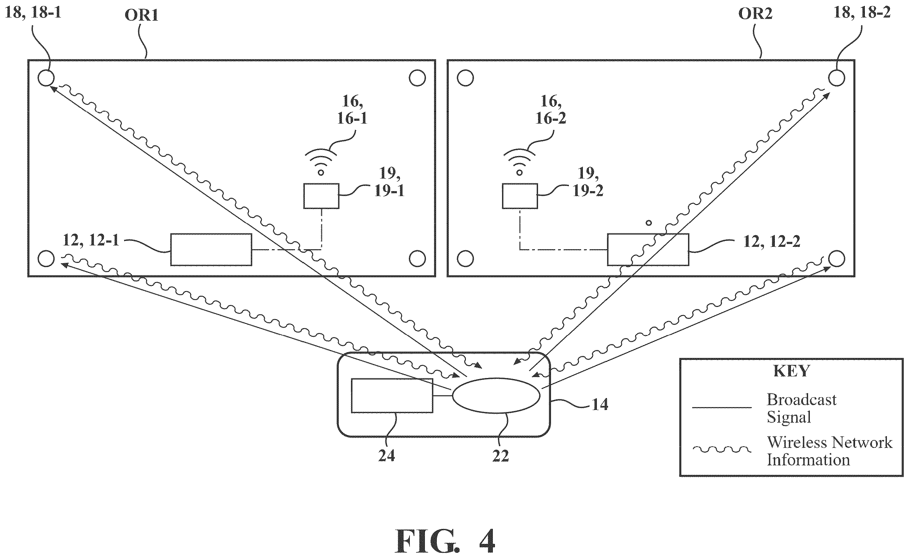

The communication system 10 may include any suitable number of operating room communication devices 18. For example, in some instances, the number of operating room communication devices 18 may correspond with the number of operating rooms OR. In the instance shown in FIGS. 1, 2A, and 2B, a first operating room communication device 18-1 is positioned in and corresponds to a first operating room OR1, and a second operating room communication device 18-2 is positioned in and corresponds to a second operating room OR2. The first operating room communication device 18-1 is configured to transmit wireless network information for connecting wirelessly with the first wireless network 16-1 and the second operating room communication device 18-2 is configured to transmit wireless network information for connecting wirelessly with the second wireless network 16-2. In other instances, more than one operating room communication device 18 may correspond with an operating room OR. In the instance shown in FIG. 4, more than one operating room communication device 18 is positioned in each of the operating rooms OR1, OR2. Specifically, four operating room communication devices 18-1 are positioned in the first operating room OR1 and four operating room communication devices 18-2 are positioned in the second operating room OR2. The four operating room communication devices 18-1 are each configured to transmit wireless network information for connecting wirelessly with the first wireless network 16-1 and the four second operating room communication device 18-2 are each configured to transmit wireless network information for connecting wirelessly with the second wireless network 16-2. As stated, the operating room communication device 18 and the medical device 14 communicate wirelessly to facilitate connection between the medical device 14 and the wireless network 16. Advantageously, a greater number of operating room communication devices 18 promotes wireless communication between the medical device 14 and an operating room communication device 18 and, thus, connection between the medical device 14 and the wireless network 16.

In some implementations, a preferred location for an operating room communication device 18 within an operating room OR may be used during placement of the operating room communication device 18 within the operating room OR. For example, a preferred location for an operating room communication device 18 within an operating room OR may be stored on a computing device (e.g. a mobile computing device and/or the hub device 12 corresponding to the operating room OR) to facilitate placement of the operating room communication device 18 within the operating room OR. Similarly, in an instance where more than one operating room communication device 18 is placed within an operating room OR, a preferred location for each of the operating room communication devices 18 may be stored on a computing device. In some instances, a user may determine a preferred location for an operating room communication device 18 within an operating room OR during setup of the operating room OR and store the location in the computing device to assist future setup procedures. In other instances, the computing device may determine a preferred location for an operating room communication device 18 within the operating room OR based on a geometry of the operating room OR and store the location in the computing device.

The operating room communication devices 18 may be positioned at any suitable location within the corresponding operating room OR. For example, referring to FIG. 1, the first operating room communication device 18-1 is attached to a wall of the first operating room OR1. Similarly, the second operating room communication device 18-2 is attached to a wall of the second operating room OR2. However, in other instances, the operating room communication devices 18 may be attached to a ceiling and/or a floor of the corresponding operating room OR. In some instances, the operating room communication devices 18 may be positioned in an operating room OR based on a preferred location and/or orientation. For example, the preferred location and/or orientation may be based on a location and/or orientation that optimizes the wireless communication between the operating room communication devices 18 and the medical device 14. For instance, the preferred location and/or orientation may be based on the range of wireless communication between the medical device 14 and the operating room communication device 18.

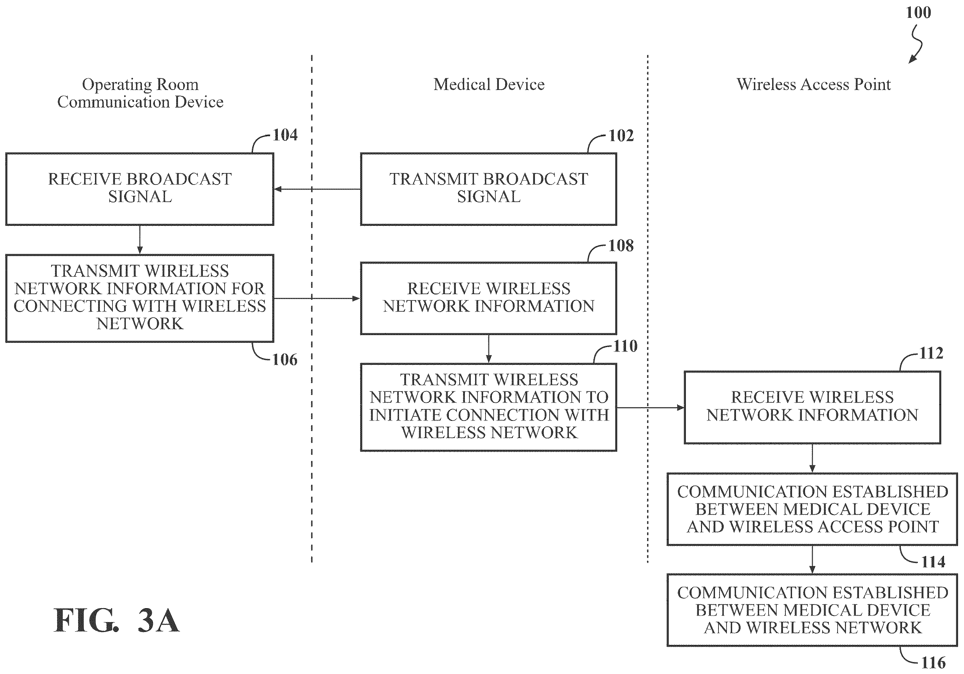

The method 100 shown in FIG. 3A further illustrates the communication between the operating room communication device 18 and the medical device 14. As shown, during step 102, the medical device 14 transmits a broadcast signal; during step 104, the operating room communication device 18 receives the broadcast signal; during step 106, the operating room communication device 18 transmits wireless network information for connecting wirelessly with the wireless network 16 in response to receiving the broadcast signal; and during step 108, the medical device 14 receives the wireless network information.

Steps 102-108 are further illustrated in FIG. 2A and 2B. As shown, the medical device 14 transmits a broadcast signal to the operating room communication devices 18-1, 18-2. In response to receiving the broadcast signal from the medical device 14, the operating room communication device 18-1 transmits the wireless network information for connecting wirelessly with the first wireless network 16-1 to the medical device 14 and the operating room communication device 18-2 transmits the wireless network information for connecting wirelessly with the second wireless network 16-2 to the medical device 14. In the instance of FIG. 4, the medical device 14 transmits a broadcast signal to the four operating room communication devices 18-1 positioned in the first operating room OR1 and to the four operating room communication devices 18-2 positioned in the second operating room OR2 (for readability purposes, the medical device 14 is shown transmitting a broadcast signal to only two of the four operating room communication devices 18-1 and to only two of the four operating room communication devices 18-2). In response to receiving the broadcast signal from the medical device 14, the four operating room communication devices 18-1 positioned in the first operating room OR1 transmit the wireless network information for connecting wirelessly with the first wireless network 16-1 to the medical device 14 and the four operating room communication devices 18-2 positioned in the second operating room OR2 transmit the wireless network information for connecting wirelessly with the second wireless network 16-2 to the medical device 14 (for readability purposes, only two of the four operating room communication devices 18-1 and only two of the four operating room communication devices 18-2 are shown transmitting wireless network information to the medical device 14).

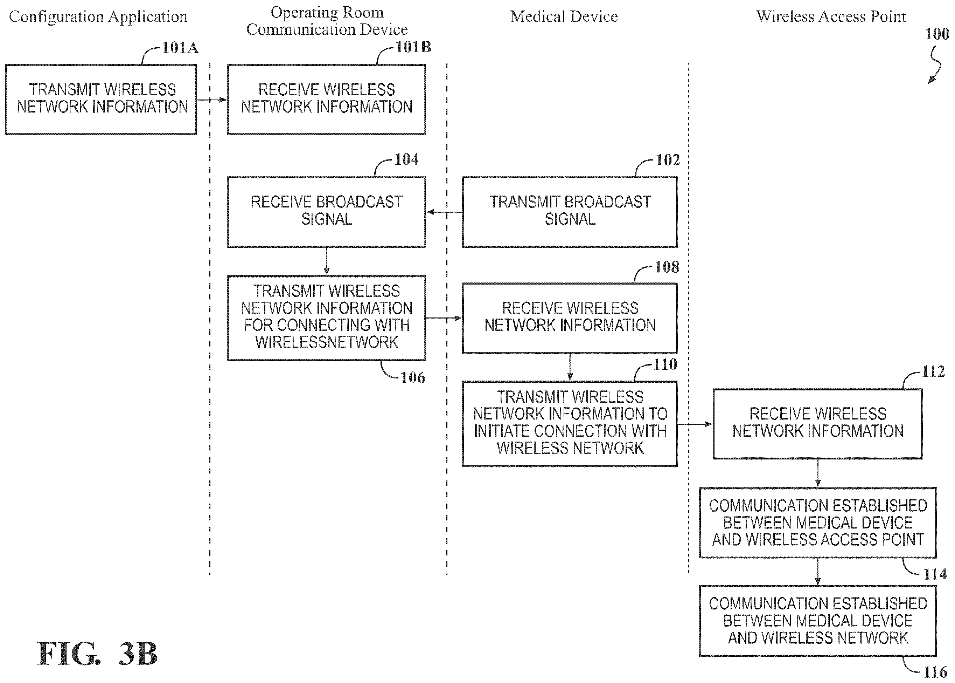

In some instances, such as the instance of FIG. 3B, the method 100 further includes step 101A of transmitting, with a configuration application, the wireless network information and a step 101B of receiving, with the operating room communication device 18, the wireless network information. Referring to FIG. 3B, in such instances, the communication system 10 includes a configuration application 15. The configuration application 15 may include a transmitter 21, which may be any suitable transmitter configured to transmit wireless network information to the operating room communication device 18. The transmitter 21 may be configured to transmit the wireless network information using any suitable communication protocol. The transmitter 21 may support any suitable wireless communication protocol, including but not limited to Bluetooth, Wi-Fi, NFC, Zigbee, Ultra-Wideband (UWB), or other short-range or long-range technologies, enabling flexible pairing and configuration across diverse device ecosystems.

The transmitter 21 may transmit the wireless network information by transmitting configuration information, which may include the wireless network information. For example, the configuration information may include the wireless network information, as well as details such as the physical location of the operating room communication device 18 (for example, coordinates or room identifiers). The configuration information may also include details of other nearby operating room communication devices 18. Additionally, the transmitter 21 may transmit updated configuration parameters to operating room communication device 18, further enabling secure communication between the medical device 14 and the wireless network 16 and accurate localization of the operating room communication device 18. Furthermore, the transmitter 21 may be configured to transmit the wireless network information to any suitable operating room communication device 18. For example, in an instance where the transmitter 21 provides the configuration information via UWB signals, a controller 17 of the configuration application 15 may use time-of-flight information of the UWB signals to locate and provide the configuration information to nearby operating room communication devices 18. Advantageously, transmission of the configuration information collectively simplifies deployment, enhances security, and supports the maintenance and scalability of networks including operating room communication devices 18.

The configuration application 15 may include a controller 17, which may include any suitable processor for processing data and any suitable memory for storing the configuration information. For example, the controller 17 may include embedded processors within a wireless-enabled device, mobile or edge processors such as ARM or x86 architectures, or remote server/cloud processors for centralized management. The controller 17 may include volatile and non-volatile storage, such as RAM, flash memory, or secure key stores, to maintain device identities, network parameters, and configuration histories. In some implementations, the controller 17 may also utilize local databases or key-value stores for offline operation and synchronize with remote databases for fleet-wide updates. Security features such as encryption of data at rest and in transit, secure credential storage, and audit logging may be employed to provide integrity and compliance across deployment models.

The configuration application 15 may include a user interface configured to receive a user input from an operator. Through this interface, the operator can provide initial configuration information, such as device identifiers, network credentials, and location-specific parameters, or modify existing settings to update the configuration information. For example, an operator may interact with the user interface of the configuration application 15 to provide and/or modify an in-range and out-range of an operating room communication device 18 (to be described in greater detail below). The user interface may also present options for selecting target devices, initiating secure pairing, and applying configuration templates, as well as advanced controls for editing wireless protocol settings, encryption preferences, and access permissions. In some implementations, the user interface may support manual entry, file-based imports, or guided workflows to simplify complex configurations. Additionally, the operator may review status indicators, confirm applied changes, and trigger rollback or version updates, supporting accuracy, security, and alignment of the configuration information with system requirements.

Once the medical device 14 receives the wireless network information for connecting wirelessly with the wireless network 16, the medical device 14 may initiate connection with the wireless network 16 by transmitting the wireless network information. As previously stated, the communication system 10 may include a wireless access point 19 configured to serve as a gateway for connecting the medical device 14 to the wireless network 16. In such instances, the wireless network information received from the operating room communication device 18 may include pairing information for initiating connection with the wireless access point 19. The medical device 14 may transmit the pairing information to the wireless access point 19 to initiate connection with the wireless access point 19 and connect wirelessly with the wireless network 16. For example, referring to FIG. 2A and 2B, the wireless network information received from the first operating room communication device 18-1 may include pairing information for initiating connection with the first wireless access point 19-1 and the wireless network information received from the second operating room communication device 18-2 may include pairing information for initiating connection with the second wireless access point 19-2. The medical device may transmit the pairing information for initiating connection with the first wireless access point 19-1 to the first wireless access point 19-1 to initiate connection with the first wireless access point 19-1 and wirelessly connect with the first wireless network 16-1. Similarly, the medical device may transmit the pairing information for initiating connection with the second wireless access point 19-2 to the second wireless access point 19-2 to initiate connection with the second wireless access point 19-2 and wirelessly connect with the second wireless network 16-2. Also as previously stated, the hub device 12 may serve as a gateway for connecting the medical device 14 to the wireless network 16 as an alternative to, or in addition to, the wireless access point 19. In such instances, the wireless network information received from the operating room communication device 18 may include pairing information for initiating connection with the hub device 12. Accordingly, the medical device 14 may transmit the pairing information to the hub device 12 to initiate connection with the hub device 12 and wirelessly connect with the wireless network 16.

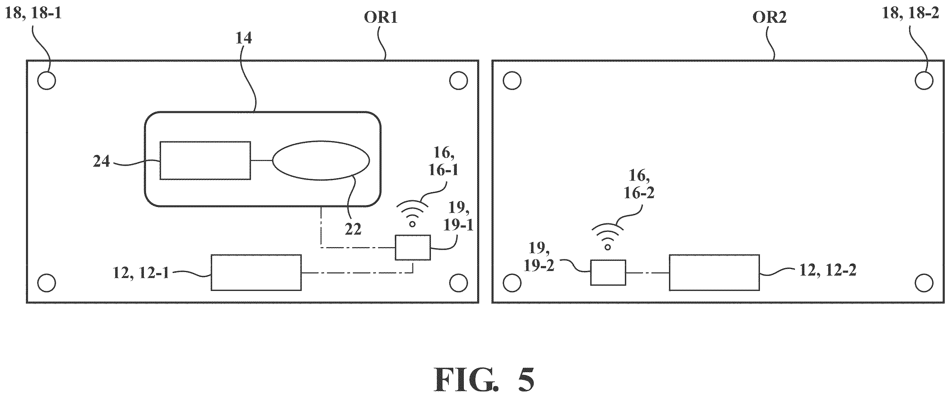

In the first implementation shown in FIGS. 1-6B, the medical device 14 initiates connection with a wireless access point 19. Referring to FIG. 3A, during step 110, the medical device 14 may transmit wireless network information to the wireless access point 19 to initiate connection with the wireless network 16; during step 112, the wireless access point 19 receives the wireless network information; during step 114, communication is established between the medical device 14 and the wireless access point 19; and during step 116, communication is established between the medical device 14 and the wireless network 16. Referring to FIG. 5, the medical device 14 is shown in communication with the wireless access point 19 and in communication with the wireless network 16.

The medical device 14 may include a controller 24 and a transmitter 22, the controller being configured to control the transmitter 22. The controller 24 may control the transmitter 22 to transmit the broadcast signal during step 102. For example, the controller 24 may receive a user input from the operator of the medical device 14 via a user interface and transmit the broadcast signal in response to receiving the user input.

The controller 24 of the medical device 14 may also control the transmitter 22 to transmit wireless network information for connecting wirelessly with the wireless network 16 during step 110.

For example, in an instance where the operating room communication device 18 transmits the wireless network information using an ultra-wideband (UWB) signal, the controller 24 of the medical device 14 may determine a time-of-flight of the received UWB signal and control the transmitter 22 to transmit the wireless network information to initiate connection with a wireless network 16 based on the time-of-flight of the received UWB signal.

For instance, the controller 24 may determine a distance between the operating room communication device 18 and the medical device 14 based on the time-of-flight of the UWB signal. The controller 24 may control the transmitter 22 to transmit the wireless network information and initiate connection with the wireless network 16 in response to determining that the distance is within the predetermined distance threshold. In some instances, the predetermined distance threshold may be determined such that the medical device 14 has entered the operating room OR when the operating room communication device 18 is within the predetermined distance threshold. In some instances, the controller 24 may provide the distance between the operating room communication device 18 and the medical device 14 to an operator of the medical device 14 via a display and transmit the wireless network information in response to receiving a user input from the operator via a user interface.

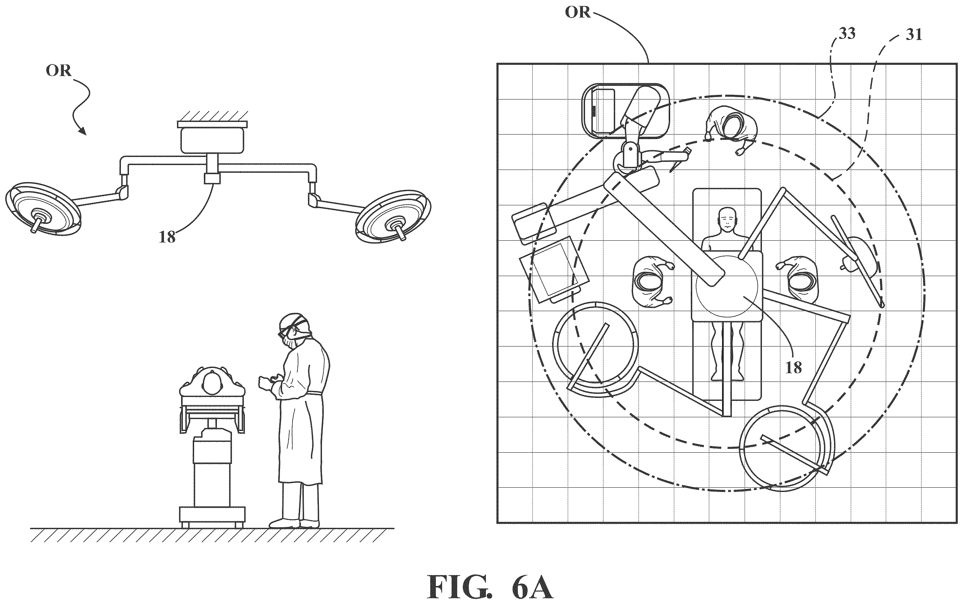

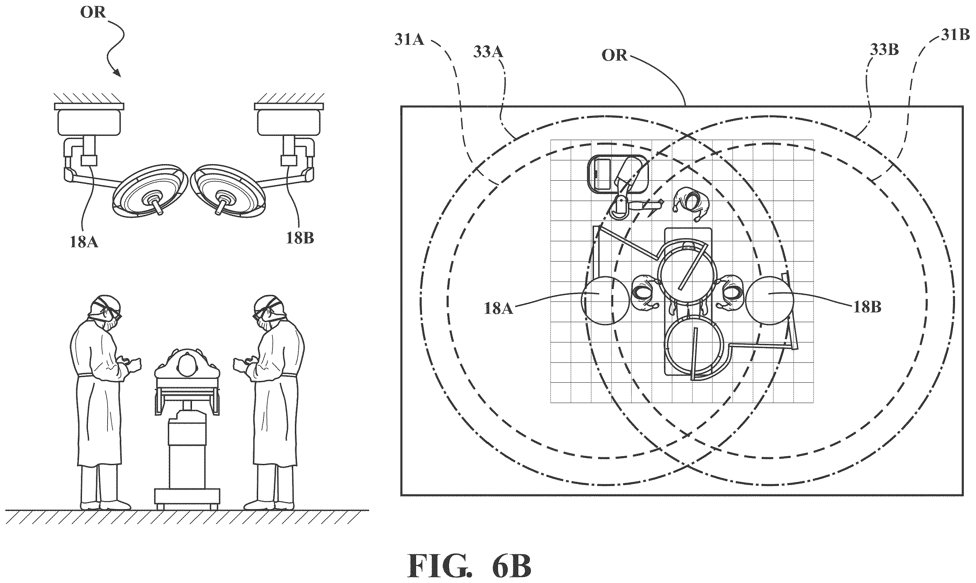

As another example, the wireless network information transmitted by an operating room communication device 18 may include an in-range of the operating room communication device 18, where the in-range may be a distance measurement (e.g. 5 feet, 10 feet, etc.). Such an instance is shown in FIGS. 6A and 6B. In such instances, the controller 24 may determine a distance between the operating room communication device 18 and the medical device 14 based on the time-of-flight of the UWB signal to determine whether the medical device 14 is within the in-range 31 of the operating room communication device 18. The controller 24 may then control the transmitter 22 to transmit the wireless network information and initiate connection with the wireless network 16 in response to determining that the medical device 14 is within the in-range 31 of the operating room communication device 18. Similarly, the controller 24 may control the transmitter 22 to not transmit the wireless network information in response to determining that the medical device 14 is outside the in-range 31 of the operating room communication device 18. In some instances, the controller 24 may notify an operator that the medical device 14 is within the in-range 31 of the operating room communication device 18. Additionally, the controller 24 may request confirmation from an operator prior to initiating connection with the wireless network 16._

In one such instance, such as the instance of FIG. 6A, the operating room OR includes a single operating room communication device 18 having an in-range 31. In such an instance, the medical device 14 may receive the wireless network information transmitted as a UWB signal by the operating room communication device 18 and the controller 24 may determine an in-range 31 of the operating room communication device 18 from the wireless network information. The controller 24 may also determine a distance between the operating room communication device 18 and the medical device 14 based on the time-of-flight of the UWB signal transmitted by the operating room communication device 18 to determine whether the medical device 14 is within the in-range 31 of the operating room communication device 18. The controller 24 may then control the transmitter 22 to transmit the wireless network information and initiate connection with the wireless network 16 in response to determining that the medical device 14 is within the in-range 31 of the operating room communication device 18.

In one such instance, such as the instance of FIG. 6B, the operating room OR includes a first operating room communication device 18A and a second operating room communication device 18B, the first operating room communication device 18A having an in-range 31A and the second operating room communication device 18B having an in-range 31B. In such an instance, the medical device 14 may receive the wireless network information transmitted as a UWB signal by one or more of the operating room communication devices 18A, 18A and the controller 24 may determine the in-ranges 31A, 31B of the operating room communication devices 18A, 18B from the wireless network information. The controller 24 may also determine a distance between the operating room communication devices 18A, 18B and the medical device 14 based on the time-of-flight of the UWB signal transmitted by the operating room communication device 18A, 18B to determine whether the medical device 14 is within the in-ranges 31A, 31B of the operating room communication devices 18A, 18B. The controller 24 may then control the transmitter 22 to transmit the wireless network information and initiate connection with the wireless network 16 based on the medical device 14 being within the in-ranges 31A, 31B of the operating room communication devices 18A, 18B. In some instances, the controller 24 may control the transmitter 22 to transmit the wireless network information and initiate connection with the wireless network 16 based on the medical device 14 being within the in-range 31A, 31B of one of the operating room communication devices 18A, 18B. In some instances, the controller 24 may control the transmitter 22 to transmit the wireless network information and initiate connection with the wireless network 16 based on the medical device 14 being within the in-range 31A, 31B of both of the operating room communication devices 18A, 18B.

As another example, in instances where the medical device 14 receives wireless network information from more than one operating room communication device 18, the controller 24 of the medical device 14 may determine with which wireless network 16 to initiate connection. For example, referring to FIG. 2A and 2B, the medical device 14 receives wireless network information for connecting wirelessly with the first wireless network 16-1 from the first operating room communication device 18-1 and wireless network information for connecting wirelessly with the second wireless network 16-2 from the second operating room communication device 18-2.

In one such instance, the controller 24 may control the transmitter 22 to transmit the wireless network information and initiate connection with a wireless network 16 based on a number of wireless network information transmissions received from operating room communication devices 18. For example, the medical device 14 may receive wireless network information for connecting wirelessly with the first wireless network 16-1 from three of the four operating room communication devices 18-1 and the medical device 14 may receive wireless network information for connecting wirelessly with the second wireless network 16-2 from two of the four operating room communication devices 18-2. The controller 24 may then control the transmitter 22 to transmit the wireless network information for connecting wirelessly with the first wireless network 16-1, as the medical device 14 received a greater number of wireless network information transmissions from the operating room communication devices 18-1.

In a further instance where the operating room communication devices 18 transmit wireless network information using a UWB signal, the controller 24 may determine a time-of-flight of the received UWB signals and control the transmitter 22 to transmit the wireless network information and initiate connection with a wireless network 16 based on the times-of-flight of the UWB signals. For instance, the controller 24 may determine a distance between the first and second operating room communication devices 18-1, 18-2 and the medical device 14 based on the times-of-flight of the UWB signals. The controller 24 may control the transmitter 22 to transmit the wireless network information to automatically initiate connection with the first wireless network 16-1 in response to determining that the distance is within the predetermined distance threshold. In some instances, the controller 24 may provide the distances between the first and second operating room communication devices 18-1, 18-2 and the medical device 14 to an operator of the medical device 14 via a display and transmit the wireless network information to initiate connection with the first or second wireless network 16-1, 16-2 in response to receiving a user input from the operator via a user interface.

Once the medical device 14 is connected with the wireless network 16, the medical device 14 may receive information from other medical devices 14 connected with the wireless network 16. In some instances, the medical devices 14 may communicate with one another to transmit location information. In one such instance, the controller 24 of a medical device 14 may determine the location of the medical device 14 based on receiving UWB signals from operating room communication devices 18 positioned in the operating room OR. The controller 24 may then determine a time-of-flight of the UWB signals to determine distances between the medical device 14 and the operating room communication devices 18. The controller 24 may determine an estimated location of the medical device 14 based on the distances between the medical device 14 and the operating room communication devices 18. The medical device 14 may communicate with other medical device 14 connected with the wireless network 16 by providing an estimated location of the medical device 14 to the other medical devices 14 and/or by providing a notification to other medical devices 14 in instances where a collision between the medical devices 14 may occur.

An access level of a medical device 14 may be based on a location of the medical device 14 within the operating room OR. For example, the controller 24 may determine a location of the medical device 14 within an operating room OR based on time-of-flight of UWB signals and alter an access level of the medical device 14 based on the location. A medical device 14 may be allowed to transmit and/or receive information to/from a remote computing device via the wireless network 16 based on the access level. Similarly, a medical device 14 may be allowed to transmit and/or receive information to/from other medical devices 14 connected with the wireless network 16 based on the access level. In one such instance, a medical device 14 may be allowed to receive a software update from a cloud server in response to being within and/or near a specified location of the operating room OR. Similarly, a medical device 14 may be allowed to transmit and/or receive information to/from other medical devices 14 in response to being within and/or near a specified of the operating room OR.

The medical device 14 may be configured to terminate connection with the wireless network 16. For example, the controller 24 of the medical device 14 may terminate connection between the medical device 14 and the wireless access point 19 to terminate connection between the medical device 14 and the wireless network 16. In one such instance, an operator may provide a user input to a user interface of the medical device 14 indicating a desire to terminate connection between the medical device 14 and the wireless network 16.

In some instances, the controller 24 of the medical device 14 may automatically terminate connection between the medical device 14 and the wireless network 16 in response to determining that the medical device 14 has exited the operating room OR corresponding to the wireless network 16.

For example, in an instance where the operating room communication device 18 transmits information via a UWB signal, the controller 24 of the medical device 14 may determine a time-of-flight of the received UWB signal and control the transmitter 22 to terminate connection with a wireless network 16 based on the time-of-flight of the received UWB signal. For instance, the controller 24 may determine a distance between the operating room communication device 18 and the medical device 14 based on the time-of-flight of the UWB signal. The controller 24 may control the transmitter 22 to automatically terminate connection with the wireless network 16 in response to determining that the distance is greater the predetermined distance threshold, where the predetermined distance threshold may be determined based on a size of the operating room OR.

As another example, the wireless network information transmitted by an operating room communication device 18 may include an out-range 33 of the operating room communication device 18, where the out-range 33 may be a distance measurement (e.g. 5 feet, 10 feet, etc.). Such an instance is shown in FIGS. 6A and 6B. In such instances, the controller 24 may determine a distance between the operating room communication device 18 and the medical device 14 based on the time-of-flight of the UWB signal to determine whether the medical device 14 is outside the out-range 33 of the operating room communication device 18. The controller 24 may then control the transmitter 22 to automatically terminate connection with the wireless network 16 in response to determining that the medical device 14 is outside the out-range 33 of the operating room communication device 18. In some instances, the controller 24 may notify an operator that the medical device 14 is outside the out-range 33 of the operating room communication device 18. Additionally, the controller 24 may request confirmation from an operator prior to terminating connection with the wireless network 16._

In one such instance, such as the instance of FIG. 6A, the operating room OR includes a single operating room communication device 18 having an out-range 33. In such an instance, the medical device 14 may receive the wireless network information transmitted as a UWB signal by the operating room communication device 18 and the controller 24 may determine an out-range 33 of the operating room communication device 18 from the wireless network information. The controller 24 may also determine a distance between the operating room communication device 18 and the medical device 14 based on the time-of-flight of the UWB signal transmitted by the operating room communication device 18 to determine whether the medical device 14 is outside the out-range 33 of the operating room communication device 18. The controller 24 may then control the transmitter 22 to terminate connection with the wireless network 16 in response to determining that the medical device 14 is outside the out-range 33 of the operating room communication device 18.

In one such instance, such as the instance of FIG. 6B, the operating room OR includes a first operating room communication device 18A and a second operating room communication device 18B, the first operating room communication device 18A having an out-range 33A and the second operating room communication device 18B having an out-range 33B. In such an instance, the medical device 14 may receive the wireless network information transmitted as a UWB signal by one or more of the operating room communication devices 18A, 18A and the controller 24 may determine the out-ranges 33A, 33B of the operating room communication devices 18A, 18B from the wireless network information. The controller 24 may also determine a distance between the operating room communication devices 18A, 18B and the medical device 14 based on the time-of-flight of the UWB signal transmitted by the operating room communication device 18A, 18B to determine whether the medical device 14 is outside the out-ranges 33A, 33B of the operating room communication devices 18A, 18B. The controller 24 may then control the transmitter 22 to transmit the wireless network information and initiate connection with the wireless network 16 based on the medical device 14 being outside the out-ranges 33A, 33B of the operating room communication devices 18A, 18B. In some instances, the controller 24 may control the transmitter 22 to terminate connection with the wireless network 16 based on the medical device 14 being outside the out-range 33A, 33B of one of the operating room communication devices 18A, 18B. In some instances, the controller 24 may control the transmitter 22 to terminate connection with the wireless network 16 based on the medical device 14 being outside the out-range 33A, 33B of both of the operating room communication devices 18A, 18B.

Generally, the controller 24 may control the transmitter 22 of the medical device 14 to initiate connection with the wireless network 16 and to terminate connection with the wireless network 16 based on a distance between the medical device 14 and an operating room communication device 18. For example, as previously stated, the controller 24 may determine a distance between an operating room communication device 18 and the medical device 14 and control the transmitter 22 to transmit the wireless network information to automatically initiate connection with the wireless network 16 in response to determining that the distance is within the predetermined distance threshold and/or in response to determining that the medical device 14 is within an in-range of an operating room communication device 18. Also previously stated, the controller 24 may determine a distance between an operating room communication device 18 and the medical device 14 and control the transmitter 22 to automatically terminate connection with the wireless network 16 in response to determining that the distance is greater the predetermined distance threshold and/or in response to determining that the medical device 14 is outside an out-range of an operating room communication device 18. In some instances, the predetermined distance threshold and the in-range may be determined such that the operating room communication device 18 is within the predetermined distance threshold and/or the in-range when the medical device 14 has entered the operating room OR. Similarly, the predetermined distance threshold and the out-range may be determined such that the operating room communication device 18 are outside the predetermined distance threshold and/or the out-range when the medical device 14 has exited the operating room OR. The predetermined distance threshold, the in-range, and the out-range may correspond an operating room OR and be based on dimensions of the operating room OR. For example, referring to FIG. 1, the predetermined distance threshold and the in-range of the first operating room OR1 may be determined such that the medical device 14 may be automatically connected with the first wireless network 16-1 in response to entering the first operating room OR1 and automatically disconnected from the first wireless network 16-1 in response to exiting the first operating room OR1. Similarly, the predetermined distance threshold and the out-range of the second operating room OR2 may be determined such that the medical device 14 may be automatically connected with the second wireless network 16-2 in response to entering the second operating room OR2 and automatically disconnected from the second wireless network 16-2 in response to exiting the second operating room OR2. Additionally, the predetermined distance threshold, in-range, and out-range may be included in the wireless network information and/or stored on any one or more of the medical device 14 and the operating room communication device 18.

In a further instance where the operating room communication devices 18 transmit wireless network information using a UWB signal, the controller 24 may determine a time-of-flight of the received UWB signals and control the transmitter 22 to transmit the wireless network information and initiate connection with a wireless network 16 based on the times-of-flight of the UWB signals. For instance, the controller 24 may determine a distance between the first and second operating room communication devices 18-1, 18-2 and the medical device 14 based on the times-of-flight of the UWB signals. The controller 24 may control the transmitter 22 to transmit the wireless network information to automatically initiate connection with the first wireless network 16-1 in response to determining that the distance is within the predetermined distance threshold and/or in response to determining that the medica device 14 is within the in-range of the operating room communication device 18-1. In some instances, the controller 24 may provide the distances between the first and second operating room communication devices 18-1, 18-2 and the medical device 14 to an operator of the medical device 14 via a display and transmit the wireless network information to initiate connection with the first or second wireless network 16-1, 16-2 in response to receiving a user input from the operator via a user interface.

The operating room communication device 18 may operate in a sleep mode and in an active mode. In such an instance, the communication system 10 may include a power source, such as a battery and/or a wall outlet coupled to the operating room communication device 18. In the sleep mode, the power source provides a first amount of power to the operating room communication device 18 and, in the active mode, the power source provides a second amount of power to the operating room communication device 18, where the first amount of power is less than the second amount of power. For example, in the sleep mode, the power source may provide enough power to the operating room communication device 18 for the operating room communication device 18 to receive the broadcast signal transmitted by the medical device 14. In the active mode, the power source may provide enough power to the operating room communication device 18 for the operating room communication device 18 to transmit the wireless network information to the medical device 14.

In some instances, the operating room communication device 18 may transition from the sleep mode to the active mode in response to receiving the broadcast signal from the medical device 14. Furthermore, the operating room communication device 18 may transmit the wireless network information to the medical device 14 in response to the operating room communication device 18 transitioning from the sleep mode to the active mode.

In some instances, the operating room communication device 18 may transition from the active mode to the sleep mode in response to receiving a sleep signal from the medical device 14. For example, the medical device 14 may be configured to transmit a sleep signal to the operating room communication device 18 in response to the medical device 14 being connected with the wireless network 16. Additionally, or alternatively, the medical device 14 may be configured to transmit a sleep signal to the operating room communication device 18 in response to termination of connection with the wireless network 16.

In some instances, the operating room communication device 18 may automatically transition from the active mode to the sleep mode based on a predetermined amount of time. For example, the operating room communication device 18 may automatically transition from the active mode to the sleep mode after a predetermined amount of time has passed since the operating room communication device 18 has received a transmission from the medical device 14. For instance, the operating room communication device 18 may automatically transition from the active mode to the sleep mode after a predetermined amount of time has passed since the operating room communication device 18 receives the broadcast signal from the medical device 14.

As previously stated, the communication system 10 may include a hub device 12. As follows, once the medical device 14 is connected with the hub device 12, the medical device 14 may communicate with the hub device 12 to receive a control signal from the hub device 12, to receive/transmit information, and/or to receive a software update from the hub device 12.

In some instances, the medical device 14 may be directly paired with an operating room communication device 18. In such instances UWB signals may be transmitted between the medical device 14 and the operating room communication device 18 to determine a location of the medical device 14 within an operating room OR. For example, time-of-flight information of the UWB signals may be used to determine the location of the medical device 14 within the operating room OR. In a further instance, time-of-flight information of the UWB signals may be compared with the in-range 31 and the out-range 33 to determine whether to continue pairing of the medical device 14 and the operating room communication device 18 (allowing continued transmission of UWB signals) to whether to terminate pairing of the medical device 14 and the operating room communication device 18 (ceasing transmission of UWB signals). More particularly, if the time-of-flight information of the UWB signals shows that the medical device 14 is within the in-range 31, pairing between the medical device 14 and the operating room communication device 18 is continued. If the time-of-flight information of the UWB signals shows that the medical device 14 is outside the out-range 33, pairing between the medical device 14 and the operating room communication device 18 is terminated (e.g. by the operating room communication device 18 and/or the controller 24 of the medical device 14). In some instances, if pairing between the medical device 14 and the operating room communication device 18 is continued, the medical device 14 continues connection with the wireless network 16. In some instances, if pairing between the medical device 14 and the operating room communication device 18 is continued, connection between the wireless network 16 and the medical device 14 is terminated.

### III. COMMUNICATION SYSTEM (SECOND IMPLEMENTATION)

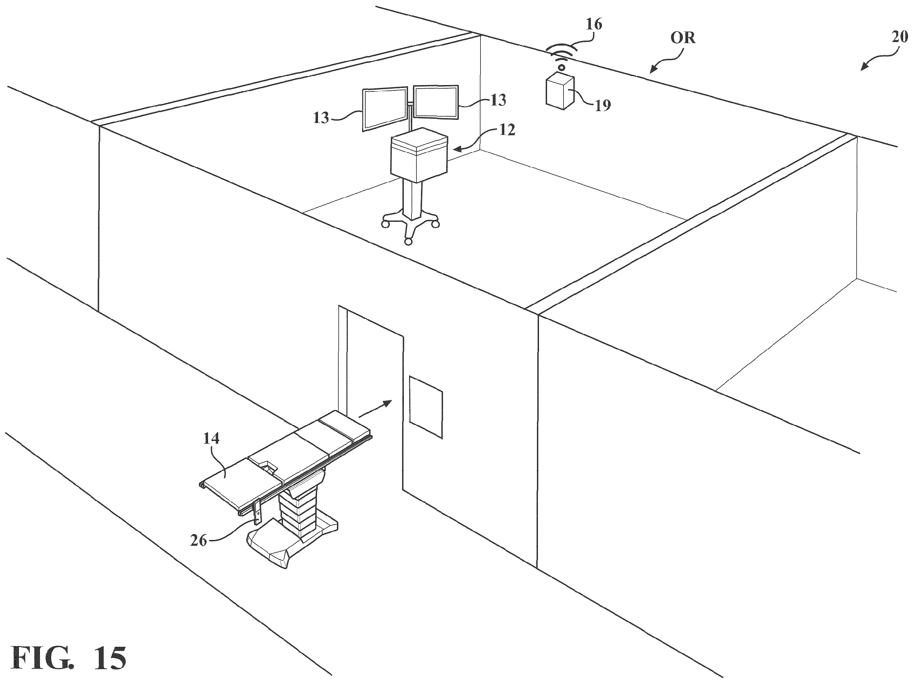

The second implementation of the communication system 10 is shown in FIGS. 7-17. In the second implementation of the communication system 10, the medical device 14 is connected with a wireless network 16 of an operating room OR. A first instance of the second implementation is shown in FIGS. 7-14 and a second instance of the second implementation is shown in FIGS. 15-17.

In the second implementation of the communication system 10, the communication system 10 includes a control device 26. The control device 26 facilitates connection between the medical device 14 and the wireless network 16. Generally, the control device 26 transmits wireless network information for connecting wirelessly with the wireless network 16. The medical device 14 receives the wireless network information and may connect with the wireless network 16 based on the wireless network information. For example, the hub device 12 may transmit wireless network information to the control device 26 and the medical device 14 may connect with the wireless network 16 using the wireless network information.

The control device 26 facilitates the connection between the medical device 14 and the wireless network 16 by transmitting wireless network information to the medical device 14. Generally, the control device 26 may be located with the medical device 14 or within the operating room OR prior to facilitating connection between the medical device 14 and the wireless network 16. In instances where the control device 26 is located within the operating room OR prior to facilitating connection, such as the first instance of FIGS. 7-14, the control device 26 may have received the wireless network information such that the control device 26 may transmit the wireless network information to the medical device 14. In instances where the control device 26 is located with the medical device 14 prior to facilitating connection, such as the second instance of FIGS. 15-17, the control device 26 receives the wireless network information and transmits the wireless network information to the medical device 14. In both instances, a device, such as the hub device 12, the configuration application, and/or an operating room communication device 18, may transmit the wireless network information to the control device 26 using any suitable communication protocol, the communication protocol including, but not limited to, wired technologies such as Ethernet, USB, and 1-Wire and wireless technologies such as Bluetooth, Wi-Fi, NFC, Zigbee, Ultra-Wideband (UWB), or other short-range or long-range technologies.

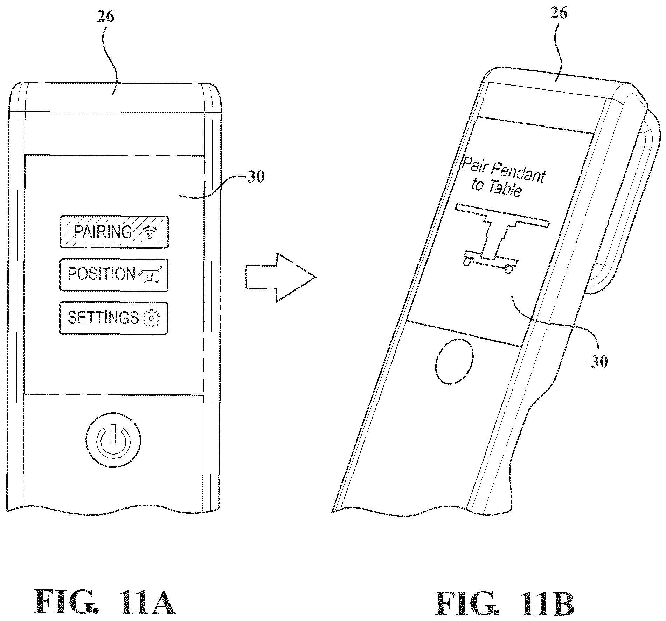

The control device 26 may include any user interface suitable for receiving a user input. For example, the control device 26 may include one or more touchscreen displays 30 (shown in FIGS. 11A and 11B) configured to receive a user input from an operator of the communication system 10. As another example, the user interface may include a trigger, a button, a directional pad, and/or a user-actuatable switch. As yet another example, the user interface may include any computing device, such as a computer, a tablet device, a smartphone device, and/or a head-mounted display device for receiving a user input. Additionally, the control device 26 may include an output device configured to provide an output to an operator of the communication system 10. For example, the control device 26 may include a display for providing visual information to the operator, such as a touchscreen display and/or a head-mounted display. As another example, the control device 26 may include light emitting diodes (LEDs) configured to emit light, a speaker for providing an audible notification, and/or a vibratory device configured to provide tactile feedback.

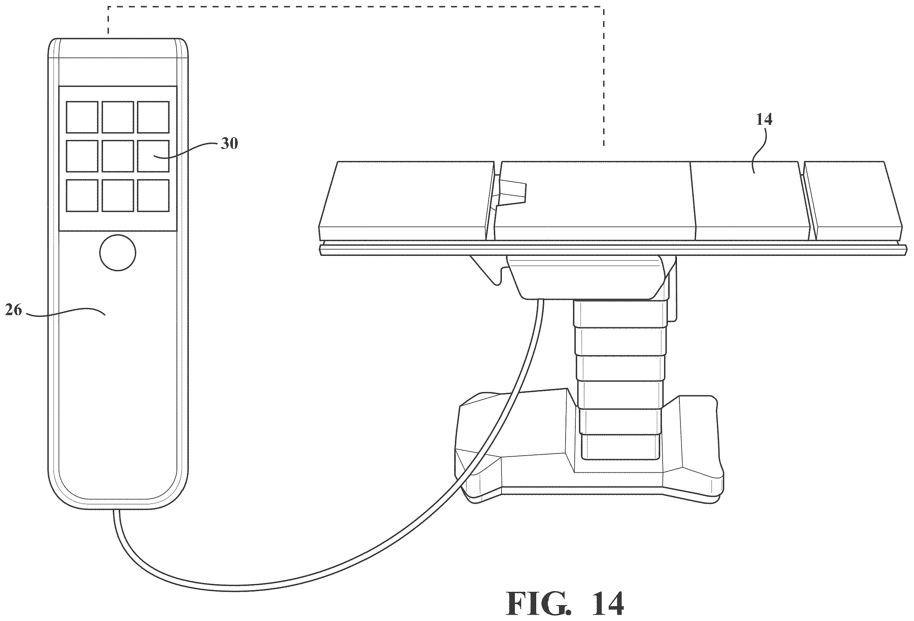

In addition to facilitating connection between the medical device 14 and the wireless network 16, the control device 26 may be configured to control the medical device 14. More particularly, the control device 26 may be configured to transmit a control signal to the medical device 14 and the medical device 14 may perform a function in response to receiving the control signal transmitted by the control device 26. For example, the touchscreen display 30 of the control device 24 may receive a user input and the control device 26 may transmit a control signal corresponding to the user input to the medical device 14. As shown in FIG. 14, the control device 26 may be in wired or wireless communication with the medical device 14 and the control device 26 may transmit the control signal via wired or wireless communication.

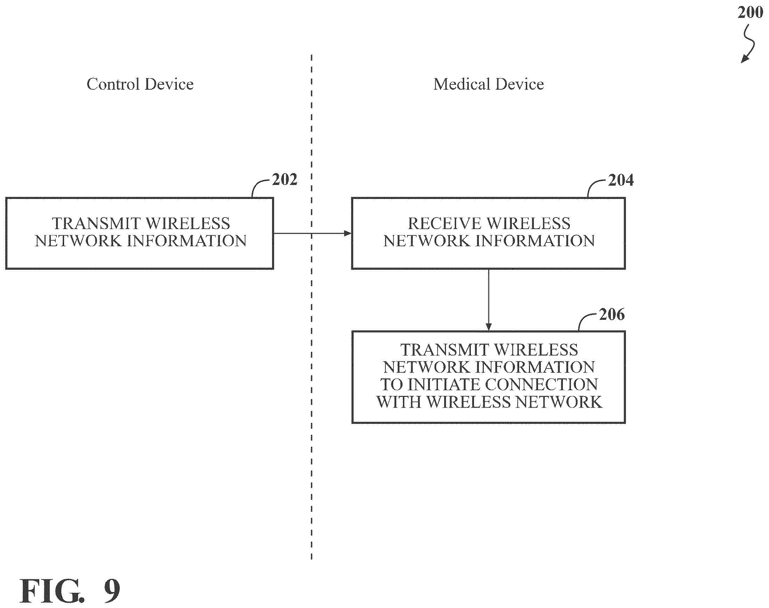

FIG. 9 illustrates a method 200 of facilitating connection between the medical device 14 and the wireless network 16 for the first instance of FIGS. 7-14. As previously stated, in the first instance, the control device 26 is located within the operating room OR prior to facilitating connection between the medical device 14 and the wireless network 16. In such instances, the control device 26 may have received the wireless network information such that the control device 26 may transmit the wireless network information to the medical device 14. For example, prior to facilitating connection between the medical device 14 and the wireless network 16, the control device 26 may be located within the operating room OR and connected to the hub device 12 via a 1-Wire communication interface. In such an instance, the hub device 26 may transmit the wireless network information to the medical device 14 in response to establishing the 1-Wire communication interface with the medical device 14. As follows, during step 202, shown in FIG. 9, the control device 26 may transmit the wireless network information to the medical device 14; during step 204, the medical device 14 receives the wireless network information; and during step 206, the medical device 14 transmits the wireless network information to initiate connection with the wireless network 16.

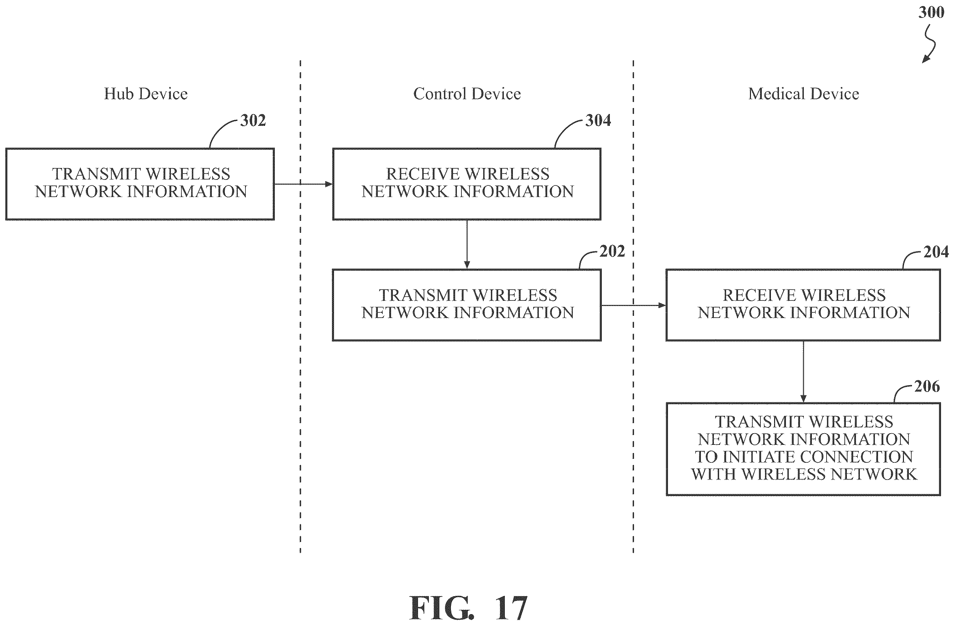

FIG. 17 illustrates a method 300 of facilitating connection between the medical device 14 and the wireless network 16 for the second instance of FIGS. 15-17. As previously stated, in the second instance, the control device 26 is located with the medical device 14 prior to facilitating connection between the medical device 14 and the wireless network 16. In such instances, the control device 26 receives wireless network information for connecting wirelessly with the wireless network 16. In the second instance, the control device 26 receives the wireless network information from the hub device 12. However, in other instances, the control device 26 may receive the wireless network information from any suitable component of the communication system 10, such as the above-described operating room communication device 18 and/or the above-described configuration application. As shown, during the step 302, the hub device 12 transmits wireless network information to the control device 26 and during step 304, the control device 26 receives the wireless network information. Additionally, the method 300 includes the steps 202-206 of the method 200 (shown in FIG. 9) such that the medical device 14 may receive the wireless network information from the control device 26 and the medical device 14 may transmit the wireless network information to initiate connection with the wireless network 16.

During steps 302 and 304 of the method 300, the hub device 12 transmits wireless network information for connecting wirelessly with the wireless network 16 to the control device 26 and the control device 26 receives the wireless network information. The hub device 12 may transmit the wireless network information to the control device 26 in a wired or wireless fashion.

For example, the hub device 12 may be configured to transmit the wireless network information in response to being electrically connected with the control device 26. In some instances, the hub device 12 may include a docking station sized to receive the control device 26, where the control device 26 electrically connects to the hub device 12 in response to the control device 26 being received by the docking station. In such instances, the hub device 12 transmits the wireless network information in response to the control device 26 being received by the docking station. The hub device 12 may be configured to transmit the wireless network information to the control device 26 using any suitable data communication protocol (e.g., wired technologies such as Ethernet, USB, and 1-Wire). In some instances, an electrical cord may be used to electrically connect the control device 26 and the hub device 12. In such instances, the hub device 12 transmits the wireless network information via the electrical cord.

As another example, the hub device 12 may be configured to wirelessly transmit the wireless network information to the control device 26. The hub device 12 and the control device 26 may communicate wirelessly using a wireless protocol. For instance, the hub device 12 and the control device 26 may be configured to communicate using near field communication (NFC) signals. The control device 26 may transmit a broadcast signal to the hub device 12 and the hub device 12 may transmit the wireless network information in response to receiving the broadcast signal. The hub device 12 and the control device 26 may be configured to communicate using any suitable communication protocol (e.g. radio, ultra-wideband, Bluetooth, near field communication, infrared, Zigbee, WiFi, cellular, and/or WiMAX communication protocols). Accordingly, a type of the transmitters, receivers, and/or transceivers of the medical device 14, the wireless access point 19, and the hub device 12 may correspond to the wireless protocol.

During steps 202 and 204, the control device 26 transmits wireless network information for connecting wirelessly with the wireless network 16 to the medical device 14. In some instances, the control device 26 transmits the wireless network information to the medical device 14 in response to being connected with the medical device 14.

In instances where the control device 26 is located with the medical device 14 prior to facilitating connection between the medical device 14 and the wireless network 16, such as the second instance of FIGS. 15-17, the control device 26 may already be connected with the medical device 14 prior to facilitating connection. In such instances, the control device 26 may transmit the wireless network information to the medical device 14 in response to receiving the wireless network information. The control device 26 may already be in wired or wireless connection with the medical device 14 prior to facilitating connection. For example, in some instances, the control device 26 may already be connected with the medical device 14 via an electrical cord. In such instances, the control device 26 may transmit the wireless network information to the medical device 14 via the electrical cord in response to receiving the wireless network information. In some instances, the control device 26 may already be wirelessly connected with the medical device 14 using a wireless pairing protocol (e.g. Bluetooth pairing). In such instances, the control device 26 may transmit the wireless network information to the medical device 14 via wireless signals in response to receiving the wireless network information.

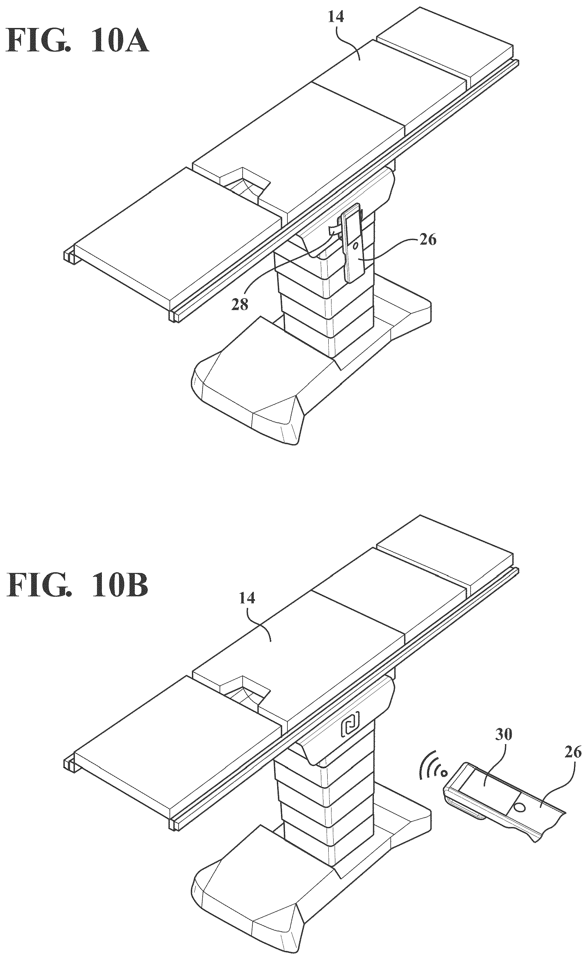

In instances where the control device 26 is located within the operating room OR prior to facilitating connection, such as the first instance of FIGS. 7-14, the control device 26 may be connected with the medical device 14 before the control device 26 transmits the wireless network information to the medical device 14. FIGS. 10A and 10B provide two examples of such a connection. In the instance of FIG. 10A, the control device 26 is electrically connected to the medical device 14 and the control device 26 transmits the wireless network information to the medical device 14 in response to the electrical connection. As shown, the medical device 14 includes a docking station 28 sized to receive the control device 26. The control device 26 electrically connects to the medical device 14 in response to the control device 26 being received by the docking station 28. In such instances, the control device 26 transmits the wireless network information in response to being received by the docking station 28. The control device 26 may be configured to transmit the wireless network information to the medical device 14 using any suitable data communication protocol (e.g., wired technologies such as Ethernet, USB, and 1-Wire). As another example, an electrical cord may be used to electrically connect the control device 26 and the medical device 14. In such instances, the control device 26 and the medical device 14 may be electrically connected via the electrical cord and the control device 26 transmits the wireless network information in response to the electrical connection. In the instance of FIG. 10B, the control device 26 transmits the wireless network information to the medical device 14 wirelessly. For example, the control device 26 may be wirelessly connected with the medical device 14 using a wireless pairing protocol (e.g. Bluetooth pairing). In such instances, the control device 26 may be wirelessly connected with the medical device 14 and the control device 26 transmits the wireless network information via wireless signals in response to the wireless connection.

FIGS. 11A and 11B illustrate an example instance where the control device 26 is connected wirelessly with the medical device 14. As shown in FIG. 11A, the touchscreen display 30 of the control device 26 may receive a user input selecting "PAIRING". The control device 26 may then be wirelessly connected to the medical device 14, as indicated in FIG. 11B, where the medical device 14 is a patient transport apparatus. Once the control device 26 and the medical device 14 are wirelessly connected, the control device 26 may transmit wireless network information to the medical device 14.

The control device 26 and the medical device 14 may include any suitable transmitters, receivers, and/or transceivers for connecting with one another. The control device 26 and the medical device 14 may be configured to connect with one another using any suitable wireless pairing protocol, including but not limited to Bluetooth, Wi-Fi, NFC, Zigbee, Ultra-Wideband (UWB), or other short-range or long-range protocols. Accordingly, a type of the transmitters, receivers, and/or transceivers of the control device 26 and the medical device 14 may correspond to the wireless protocol.

In some instances where the control device 26 is located with the medical device 14 prior to facilitating connection between the medical device 14 and the wireless network 16, the control device 26 may not be connected with the medical device 14 prior to facilitating connection. In such instances, the control device 26 may be connected with the medical device 14 in accordance with the above description and in accordance with FIGS. 10A-11B. The control device 26 may then transmit the wireless network information to the medical device 14 in response to being connected with the medical device 14.

In some instances, the control device 26 may transmit the wireless network information to the medical device 14 prior to or without being connected with the medical device 14. For example, the control device 26 may transmit the wireless network information to the medical device 14 using a near field communications (NFC) signal. The medical device 14 may receive the NFC signal and receive the wireless network information. In some instances, the control device 26 may then be connected with the medical device 14 using any suitable wireless pairing protocol. In some instances, connection between the control device 26 and the medical device 14 using a wireless pairing protocol may be omitted.

During step 206, the medical device 14 may transmit the wireless network information for connecting wirelessly with the wireless network 16 to initiate connection with the wireless network 16. More particularly, the control device 26 may receive the wireless network information during step 204 and transmit the wireless network information to initiate connection during step 206. The medical device 14 may initiate connection with the wireless network using any above-described means. For example, as previously stated, the communication system 10 may include a wireless access point 19 configured to serve as a gateway for connecting the medical device 14 to the wireless network 16. In such instances, the medical device 14 may transmit the wireless network information to the wireless access point 19 to initiate connection with the wireless access point 19 and connect wirelessly with the wireless network 16. Additionally, as previously stated, the hub device 12 may be configured to serve as a gateway for connecting the medical device 14 to the wireless network 16 as an alternative to the wireless access point 19, or in addition to the wireless access point 19. In such instances, the medical device 14 may transmit the wireless network information to the hub device 12 to initiate connection with the hub device 12 and connect wirelessly with the wireless network 16.

As previously stated, once communication is established between the medical device 14 and the wireless network 16, the medical device 14 may upload (transmit) and download (receive) information via the wireless network 16. Additionally, the medical device 14 may transmit and receive information from other devices connected with the wireless network 16.

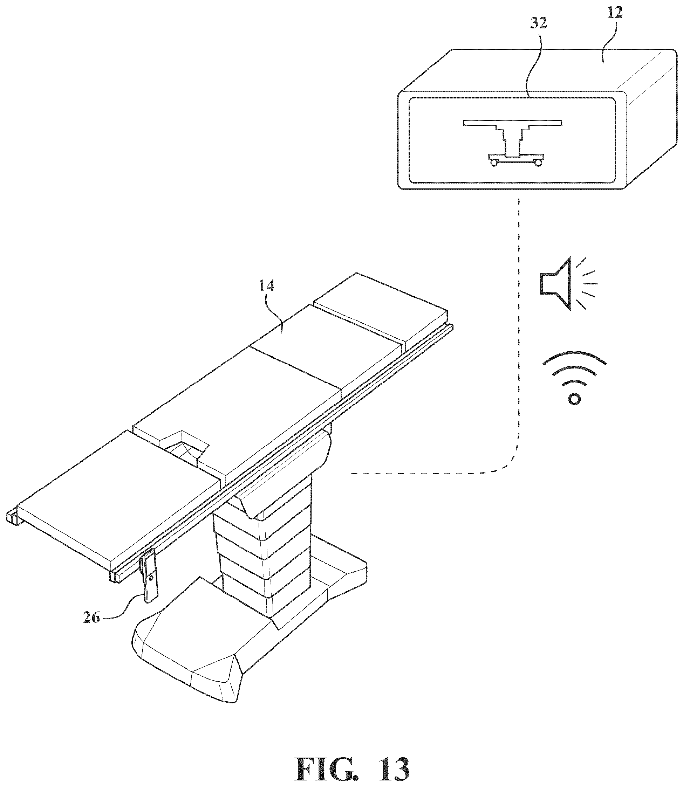

Additionally, the communication system 10 may provide a notification after the control device 26 is connected with the wireless network 16. For example, the control device 26 may provide a notification that the medical device 14 has connected with the wireless network 16. For example, the touchscreen display 30 of the control device 26 may provide a visual notification that the medical device 14 has established communication with the wireless network 16. In other instances, the control device 26 may provide audio and/or tactile feedback to indicate that the medical device 14 has established communication with the wireless network 16. In some instances, the hub device 12 and/or medical device 14 may also provide a notification that the medical device 14 has connected with the wireless network 16. The hub device 12 may include a user interface and/or display device, represented as a touchscreen display 32 in FIG. 13. The display device 32 of the hub device 12 may provide a visual and/or audio notification that the medical device 14 has established communication with the wireless network 16. Similarly, a user interface of the medical device 14 may provide a visual and/or audio notification that the medical device 14 has established communication with the wireless network 16. In other instances, the hub device 12 and/or the medical device 14 may provide tactile feedback to indicate that the medical device 14 has established communication with the wireless network 16.

Once the medical device 14 is connected with the hub device 12, the medical device 14 may communicate with the hub device 12 to receive a control signal from the hub device 12, to receive/transmit information, and/or to receive a software update from the hub device 12.

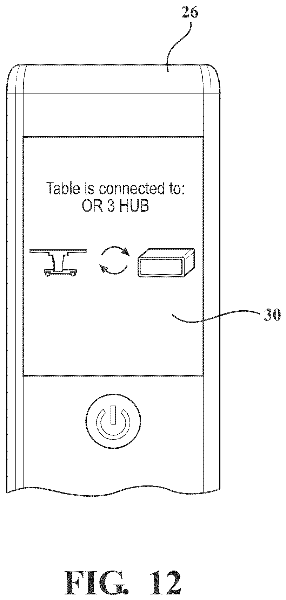

Additionally, the communication system 10 may provide a notification after the control device 26 is connected with the hub device 12. For example, referring to FIG. 12, the control device 26 may provide a notification that the medical device 14 has connected with the hub device 12. For example, the touchscreen display 30 of the control device 26 may provide a visual notification that the medical device 14 has established communication with the hub device 12. In other instances, the control device 26 may provide audio and/or tactile feedback to indicate that the medical device 14 has established communication with the hub device 12. Referring to FIG. 13, the hub device 12 and/or the medical device 14 may also provide a notification that the medical device 14 has connected with the hub device 12. The hub device 12 may include a user interface and/or display device, represented as a touchscreen display 32 in FIG. 13. The display device 32 of the hub device 14 may provide a visual and/or audio notification that the medical device 14 has established communication with the hub device 12. Similarly, a user interface of the medical device 14 may provide a visual and/or audio notification that the medical device 14 has established communication with the hub device 12. In other instances, the hub device 12 and/or the medical device 14 may provide tactile feedback to indicate that the medical device 14 has established communication with the hub device 12.

Once the medical device 14 and the hub device 12 are connected with one another, both the hub device 12 and the control device 26 may provide a control signal to the medical device 14. For example, referring to FIG. 14, the control device 26 may provide a control signal to the medical device 14 via wired or wireless communication. Additionally, the hub device 12 may provide a control signal to the medical device 14. For example, the hub device 12 may include a user interface configured to receive a user input, such as the touchscreen display 32 shown in FIG. 13. The hub device 12 may transmit the control signal to the medical device 14 based on the user input.

In some instances, in the first and second instance of the second implementation of the communication system 10, the medical device 14 may be paired with a device configured to communicate via UWB signals. For example, the communication system 10 may include one or more of the previously described operating room communication device 18. Additionally, or alternatively, the hub device 12 and/or wireless access point 19 may include functionality of the operating room communication device 18. For example, the medical device 14 may be directly paired with an operating room communication device 18. In such instances UWB signals may be transmitted between the medical device 14 and the operating room communication device 18 to determine a location of the medical device 14 within an operating room OR. For example, time-of-flight information of the UWB signals may be used to determine the location of the medical device 14 within the operating room OR. In a further instance, time-of-flight information of the UWB signals may be compared with the in-range 31 and the out-range 33 to determine whether to continue pairing of the medical device 14 and the operating room communication device 18 (allowing continued transmission of UWB signals) to whether to terminate pairing of the medical device 14 and the operating room communication device 18 (ceasing transmission of UWB signals). More particularly, if the time-of-flight information of the UWB signals shows that the medical device 14 is within the in-range 31, pairing between the medical device 14 and the operating room communication device 18 is continued. If the time-of-flight information of the UWB signals shows that the medical device 14 is outside the out-range 33, pairing between the medical device 14 and the operating room communication device 18 is terminated (e.g. by the operating room communication device 18 and/or the controller 24 of the medical device 14). In some instances, if pairing between the medical device 14 and the operating room communication device 18 is continued, the medical device 14 continues connection with the wireless network 16. In some instances, if pairing between the medical device 14 and the operating room communication device 18 is continued, connection between the wireless network 16 and the medical device 14 is terminated.

US Provisional Patent Application entitled "Systems, Devices, and Method for Establishing Wireless Communication Links", with reference numbers ENDO0719PROV and 70742-30142.00, filed on February 5, 2025, is hereby incorporated by reference in its entirety.

The present disclosure also comprises the following CLAUSES, which may be additionally accompanied by any of the aspects or implementations provided in the summary and/or by any features otherwise found in the disclosure or the figures of the subject application.

Clause A1. A method of operating a communication system, the communication system including a medical device and an operating room communication device, the method including steps of: transmitting, with the medical device, a broadcast signal; receiving, with the operating room communication device, the broadcast signal; transmitting, with the operating room communication device, wireless network information for connecting wirelessly with the wireless network in response to receiving the broadcast signal; receiving, with the medical device, the wireless network information from the operating room communication device; and transmitting, with the medical device, the wireless network information to initiate connection with the wireless network. Clause A2. The method of Clause A1, wherein the communication system further includes a wireless access point in communication with the wireless network. Clause A3. The method of Clause A2, wherein the step of transmitting, with the medical device, the wireless network information to initiate connection with the wireless network further includes a step of transmitting, with the medical device, the wireless network information to the wireless access point to initiate connection with the wireless access point. Clause A4. The method of any one of Clauses A1-A3, wherein the communication system further includes a configuration application, and further comprising steps of: transmitting, with the configuration application, the wireless network information to the operating room communication device; and receiving, with the operating room communication device, the wireless network information from the configuration application prior to transmitting, with the operating room communication device, the wireless network information. Clause A5. The method of any one of Clauses A1-A4, wherein the communication system further includes a hub device, and further comprising steps of: establishing connection between the hub device and the medical device; and transmitting, with the hub device, a control signal to the medical device. Clause A6. The method of Clause A5, wherein the step of transmitting, with the medical device, the wireless network information to initiate connection with the wireless network further includes a step of transmitting, with the medical device, the wireless network information to the hub device to initiate connection with the wireless network. Clause A7. The method of any one of Clauses A1-A6, wherein the step of transmitting, with the medical device, the broadcast signal further includes a step of transmitting, with the medical device, the broadcast signal using an ultrawide band signal, and wherein the step of transmitting, with the operating room communication device, the wireless network information further includes a step of transmitting, with the operating room communication device, the wireless network information with an ultrawide band signal. Clause A8. The method of Clause A7, wherein the medical device includes a controller and a transmitter, and further comprising steps of: determining, with the controller, a time-of-flight of the ultrawide band signal transmitted by the operating room communication device; and controlling, with the controller, the transmitter to transmit the wireless network information to initiate connection with the wireless network based on the time-of-flight of the ultrawide band signal. Clause A9. The method of Clause A8, further comprising steps of: determining, with the controller, a distance between the operating room communication device and the medical device based on the time-of-flight of the ultrawide band signal; determining, with the controller, that the distance between the operating room communication device and the medical device is within a predetermined distance threshold; and controlling, with the controller, the transmitter to transmit the wireless network information to initiate connection with the wireless network in response to determining that the distance between the operating room communication device and the medical device is within the predetermined distance threshold. Clause A10. The method of any one of Clauses A8 and A9, further comprising steps of: determining, with the controller, a time-of-flight of the ultrawide band signal transmitted by the operating room communication device; and terminating, with the controller, connection of the medical device and the wireless network based on the time-of-flight of the ultrawide band signal. Clause A11. The method of Clause A10, further comprising steps of: determining, with the controller, a distance between the operating room communication device and the medical device based on the time-of-flight of the ultrawide band signal; determining, with the controller, that the distance between the operating room communication device and the medical device is greater than a predetermined distance threshold; and terminating, with the controller, connection of the medical device and the wireless network in response to determining that the distance between the operating room communication device and the medical device is greater than the predetermined distance threshold. Clause A12. The method of Clause A11, wherein the medical device includes a controller and a transmitter, and further comprising steps of: determining, with the controller, a time-of-flight of the ultrawide band signal transmitted by the operating room communication device; determining, with the controller, a distance between the operating room communication device and the medical device based on the time-of-flight of the ultrawide band signal; determining, with the controller, that the distance between the operating room communication device and the medical device is greater than a predetermined distance threshold; and terminating, with the medical device, connection of the medical device with the wireless network in response to determining that the distance between the operating room communication device and the medical device is greater than a predetermined distance threshold.

Clause B1. A system for connecting a medical device to a wireless network comprising: a medical device; a control device in communication with the medical device, the control device being configured to transmit a control signal, wherein the medical device is configured to receive the control signal and perform a function based on the control signal transmitted by the control device; and a hub device being configured to transmit wireless network information for connecting wirelessly with the wireless network to the control device; wherein the control device is configured to transmit the wireless network information to the medical device; and wherein the medical device is configured to transmit the wireless network information to initiate connection with the wireless network. Clause B2. The system of Clause B1, further including a wireless access point in communication with the wireless network. Clause B3. The system of Clause B2, wherein the medical device is configured to transmit the wireless network information to the wireless access point to initiate connection with the wireless network. Clause B4. The system of any one of Clauses B1-B3, wherein the medical device is configured to transmit the wireless network information to the hub device to initiate connection with the wireless network. Clause B5. The system of any one of Clauses B1-B4, wherein the hub device is configured to transmit a control signal, and wherein, in response to the medical device being connected with the wireless network, the medical device is configured to receive the control signal transmitted by the hub device and perform the function based on the control signal transmitted by the hub device. Clause B6. The system of any one of Clauses B1-B5, wherein medical device is configured to transmit operational information to the hub device in response to the medical device being connected with the wireless network. Clause B7. The system of Clause B6, wherein the hub device includes a display device, the display device being configured to display the operational information. Clause B8. The system of any one of Clauses B1-B7, wherein the control device is configured to transmit a broadcast signal to the hub device using a near field communication signal, and wherein the hub device is configured to transmit the wireless network information in response to receiving the broadcast signal. Clause B9. The system of any one of Clauses B1-B8, wherein the control device and the hub device are configured to electrically connect. Clause B10. The system of Clause B9, wherein the hub device and the control device are configured to electrically connect via a 1-Wire communication interface, and wherein the hub device is configured to transmit wireless network information to the control device via the 1-Wire communication interface. Clause B11. The system of any one of Clauses B9 and B10, wherein the hub device is coupled to a docking station sized to receive the control device, and wherein the control device and the hub device are configured to electrically connect in response to the control device being received by the docking station. Clause B12. The system of any one of Clauses B9-B11, wherein the control device is configured to connect wirelessly with the medical device such that the control signal is transmitted via the wireless connection. Clause B13. The system of any one of Clauses B9-B12, wherein the control device is configured to electrically connect with the medical device such that the control signal is transmitted via the electrical connection.

Clause C1. A method of operating a communication system, the communication system including a medical device, a hub device, and a control device, the method including steps of: transmitting, with the control device, a control signal; receiving, with the medical device, the control signal transmitted by the control device; performing, with the medical device, a function based on the control signal transmitted by the control device; transmitting, with the hub device, wireless network information for connecting wirelessly with the wireless network; receiving, with the control device, the wireless network information from the hub device; transmitting, with the control device, the wireless network information to the medical device; and transmitting, with the medical device, the wireless network information to initiate connection with the wireless network. Clause C2. The method of Clause C1, wherein the communication system further includes a wireless access point in communication with the wireless network, and further comprising a step of transmitting, with the medical device, the wireless network information to the wireless access point to initiate connection with the wireless network. Clause C3. The method of any one of Clauses C1 and C2, further comprising a step of transmitting, with the medical device, the wireless network information to the hub device to initiate connection with the wireless network. Clause C4. The method of any one of Clauses C1-C3, further comprising a step of transmitting, with the control device, a broadcast signal to the hub device. Clause C5. The method of Clause C4, wherein the step of transmitting, with the hub device, wireless network information for connecting wirelessly with the wireless network comprises a step of transmitting, with the hub device, wireless network information for connecting wirelessly with the wireless network in response to receiving the broadcast signal. Clause C6. The method of any one of Clauses C1-C5, further comprising a step of electrically connecting the control device and the hub device. Clause C7. The method of any one of Clauses C1-C6, further comprising steps of: transmitting, with the hub device, a control signal; and receiving, with the medical device, the control signal transmitted by the hub device; performing, with the medical device, a function based on the control signal transmitted by the hub device in response to the medical device being connected with the wireless network. Clause C8. The method of any one of Clauses C1-C7, further comprising a step of transmitting, with the medical device, at least one of health information of the medical device and patient information in response to the medical device being connected with the wireless network.

Clause D1. A system for connecting a medical device to a wireless network comprising: a medical device configured to perform a function; a control device being configured to: transmit wireless network information to the medical device, wherein the medical device is configured to transmit the wireless network information to initiate connection with the wireless network; and transmit a control signal, wherein, in response to the control device being connected with the medical device, the medical device is configured to receive the control signal and perform the function based on the control signal. Clause D2. The system of Clause D1, further including a wireless access point in communication with the wireless network. Clause D3. The system of Clause D2, wherein the medical device is configured to transmit the wireless network information to the wireless access point to initiate connection with the wireless network. Clause D4. The system of any one of Clauses D1-D3, further comprising a hub device in communication with the wireless network. Clause D5. The system of Clause D4, wherein the medical device is configured to transmit the wireless network information to the hub device to initiate connection with the wireless network. Clause D6. The system of any one of Clauses D4 and D5, wherein the hub device is configured to transmit a control signal, and wherein, in response to the medical device being connected with the wireless network, the medical device is configured to receive the control signal transmitted by the hub device and perform the function based on the control signal transmitted by the hub device. Clause D7. The system of any one of Clauses D4-D6, wherein the medical device is configured to transmit operational information to the hub device in response to the medical device being connected with the wireless network. Clause D8. The system of Clause D7, wherein the hub device includes a display device, the display device being configured to display the operational information. Clause D9. The system of any one of Clauses D1-D8, wherein the control device is configured to transmit wireless network information to the medical device using a near field communication signal. Clause D10. The system of any one of Clauses D1-D9, wherein the control device and the medical device are configured to electrically connect. Clause D11. The system of Clause D10, wherein the control device and the medical device are configured to electrically connect via a 1-Wire communication interface, and wherein the control device is configured to transmit wireless network information to the medical device via the 1-Wire communication interface. Clause D12. The system of any one of Clauses D10 and D11, wherein the medical device is coupled to a docking station sized to receive the control device, and wherein the control device and the medical device are configured to electrically connect in response to the control device being received by the docking station. Clause D13. The system of any one of Clauses D1-D12, wherein the control device is configured to connect wirelessly with the medical device such that the control signal is transmitted via the wireless connection.

Clause E1. A method of operating a communication system, the communication system including a medical device and a control device, the method including steps of: transmitting, with the control device, wireless network information to the medical device in response to the medical device being connected with the control device; transmitting, with the medical device, the wireless network information to initiate connection with the wireless network; transmitting, with the control device, a control signal; receiving, with the medical device, the control signal transmitted by the control device; and performing, with the medical device, a function in response to the medical device being connected with the control device based on the control signal transmitted by the control device. Clause E2. The method of Clause E1, wherein the communication system further includes a wireless access point in communication with the wireless network, and further comprising a step of transmitting, with the medical device, the wireless network information to the wireless access point to initiate connection with the wireless network. Clause E3. The method of any one of Clauses E1 and E2, wherein the communication system further includes a hub device in communication with the wireless network, and further comprising steps of: transmitting, with the medical device, the wireless network information to the hub device to initiate connection with the wireless network; transmitting, with the hub device, a control signal; receiving, with the medical device, the control signal transmitted by the hub device; and performing, with the medical device, a function based on the control signal transmitted by the hub device in response to the medical device being connected with the wireless network. Clause E4. The method of any one of Clauses E1-E3, further comprising a step of transmitting, with the medical device, at least one of health information of the medical device and patient information in response to the medical device being connected with the wireless network. Clause E5. The method of any one of Clauses E1-E4, further comprising a step of transmitting, with the control device, wireless network information to the medical device using a near field communication signal. Clause E6. The method of Clause E3, further comprising a step of electrically connecting the control device and the hub device.

Clause F1. A system for connecting a medical device with a wireless network including: a configuration application configured to transmit wireless network information; an operating room communication device configured to: receive a broadcast signal; receive the wireless network information from the configuration application; and transmit the wireless network information for connecting wirelessly with the wireless network in response to receiving the broadcast signal; and a medical device configured to: transmit the broadcast signal to the operating room communication device; receive wireless network information from the operating room communication device; and transmit the wireless network information to initiate connection with the wireless network.

Clause G1. A system for connecting a medical device with a wireless network including: an operating room communication device configured to: receive a broadcast signal; and transmit wireless network information using an ultrawide band signal for connecting wirelessly with the wireless network in response to receiving the broadcast signal; and a medical device configured to: transmit the broadcast signal to the operating room communication device using an ultrawide band signal; receive wireless network information from the operating room communication device; determine a time-of-flight of the ultrawide band signal transmitted by the operating room communication device; determine a distance between the operating room communication device and the medical device based on the time-of-flight of the ultrawide band signal; determine that the distance between the operating room communication device and the medical device is within a predetermined distance threshold; and transmit the wireless network information to initiate connection with the wireless network in response to determining that the distance between the operating room communication device and the medical device is within the predetermined distance threshold.

Clause H1. A system for connecting a medical device to a wireless network comprising: a medical device; a control device in communication with the medical device, the control device being configured to transmit a control signal, wherein the medical device is configured to receive the control signal and perform a function based on the control signal transmitted by the control device; and a hub device configured to electrically connect with the control device via a 1-Wire communication interface, the hub device being configured to transmit wireless network information for connecting wirelessly with the wireless network to the control device via the 1-Wire communication device; wherein the control device is configured to transmit the wireless network information to the medical device; and wherein the medical device is configured to transmit the wireless network information to initiate connection with the wireless network.

Clause I1. A system for connecting a medical device to a wireless network comprising: a medical device configured to perform a function; a control device being configured to: electrically connect with the medical device via a 1-Wire communication interface; transmit wireless network information to the medical device via the 1-Wire communication interface, wherein the medical device is configured to transmit the wireless network information to initiate connection with the wireless network; and transmit a control signal, wherein, in response to the control device being connected with the medical device, the medical device is configured to receive the control signal and perform the function based on the control signal.

Several embodiments have been discussed in the foregoing description. However, the embodiments discussed herein are not intended to be exhaustive or limit the invention to any particular form. The terminology which has been used is intended to be in the nature of words of description rather than of limitation. Many modifications and variations are possible in light of the above teachings and the invention may be practiced otherwise than as specifically described.

The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, all suitable modifications and equivalents may be resorted to, falling within the scope of the invention.

## Claims

1. A system (10) for connecting a medical device (14) with a wireless network (16) including:
an operating room communication device (18) configured to:
receive a broadcast signal; and
transmit wireless network information for connecting wirelessly with the wireless network (16) in response to receiving the broadcast signal; and
a medical device (14) configured to:
transmit the broadcast signal to the operating room communication device (18);
receive wireless network information from the operating room communication device (18); and
transmit the wireless network information to initiate connection with the wireless network (16).

2. The system (10) of claim 1, further including a wireless access point (19) in communication with the wireless network (16), wherein the medical device (14) is configured to transmit the wireless network information to the wireless access point (19) to initiate connection with the wireless network (16), and wherein the wireless network information includes pairing information for initiating connection with the wireless access point (19).

3. The system (10) of claim 1 or 2, further including a hub device (12) configured to connect with the medical device (14) and transmit a control signal to the medical device (14) after the hub device (12) has connected with the medical device (14), wherein the medical device (14) is configured to transmit the wireless network information to the hub device (12) to initiate connection with the wireless network (16), and wherein the wireless network information includes pairing information for initiating connection with the hub device (12).

4. The system (10) of any one of claims 1 to 3, further comprising a configuration application (15) configured to transmit the wireless network information to the operating room communication device (18), and wherein the operating room communication device (18) is configured to receive the wireless network information from the configuration application (15) prior to transmitting the wireless network information.

5. The system (10) of any one of claims 1 to 4, further including a power source coupled to the operating room communication device (18), and wherein:
the operating room communication device (18) is configured to operate in:
a sleep mode where the power source provides a first amount of power to the operating room communication device (18); and
an active mode where the power source provides a second amount of power to the operating room communication device (18), where the first amount of power is less than the second amount of power.

6. The system (10) of claim 5, wherein, in response to receiving the broadcast signal, the operating room communication device (18) is configured to transition from the sleep mode to the active mode, and wherein the operating room communication device (18) is configured to transmit the wireless network information to the medical device (14) in response to the operating room communication device (18) transitioning from the sleep mode to the active mode.

7. The system (10) of claim 5 or 6, wherein the medical device (14) is configured to transmit a sleep signal to the operating room communication device (18) in response to the medical device (14) being connected with the wireless network (16), and wherein the operating room communication device (18) is configured to transition from the active mode to the sleep mode in response to receiving the sleep signal.

8. The system (10) of any one of claims 1 to 7, wherein the medical device (14) is configured to transmit the broadcast signal using an ultrawide band signal, and wherein the operating room communication device (18) is configured to transmit wireless network information using an ultrawide band signal.

9. The system (10) of claim 8, wherein the medical device (14) includes a controller (24) and a transmitter (22), wherein the controller (24) is configured to:
determine a time-of-flight of the ultrawide band signal transmitted by the operating room communication device (18);
determine a distance between the operating room communication device (18) and the medical device (14) based on the time-of-flight of the ultrawide band signal;
determine that the distance between the operating room communication device (18) and the medical device (14) is within a predetermined distance threshold; and
control the transmitter (22) to transmit the wireless network information to initiate connection with the wireless network (16) in response to determining that the distance between the operating room communication device (18) and the medical device (14) is within the predetermined distance threshold.

10. The system (10) of claim 8, wherein the medical device (14) includes a controller (24) and a transmitter (22), wherein the controller (24) is configured to:
determine a time-of-flight of the ultrawide band signal transmitted by the operating room communication device (18);
determine a distance between the operating room communication device (18) and the medical device (14) based on the time-of-flight of the ultrawide band signal;
determine that the distance between the operating room communication device (18) and the medical device (14) is greater than a predetermined distance threshold; and
terminate connection of the medical device (14) and the wireless network (16) in response to determining that the distance between the operating room communication device (18) and the medical device (14) is greater than the predetermined distance threshold.

11. The system (10) of any one of claims 1 to 10, wherein the wireless network (16) is further defined as a first wireless network (16), wherein the operating room communication device (18) is further defined as a first operating room communication device (18), and further including a second operating room communication device (18) configured to:
receive the broadcast signal; and
transmit wireless network information for connecting wirelessly with a second wireless network (16) in response to receiving the broadcast signal.

12. The system (10) of claim 11, wherein the medical device (14) includes a controller (24) and a transmitter (22), wherein the controller (24) is configured to:
control the transmitter (22) to transmit the wireless network information for connecting wirelessly with the first wireless network to initiate connection with the first wireless network; and
control the transmitter (22) to transmit the wireless network information for connecting wirelessly with the second wireless network (16) to initiate connection with the second wireless network (16).

13. The system (10) of claim 12, wherein the medical device (14) includes a user interface configured to receive a user input, and wherein the controller (24) is configured to control the transmitter to transmit the wireless network information for connecting wirelessly with the first wireless network (16) or the wireless network information for connecting wirelessly with the second wireless network (16) based on the user input.

14. The system (10) of any one of claims 1 to 13, wherein the medical device (14) is further defined as a first medical device (14), and further including a second medical device (14) connected with the wireless network (16), and wherein the first medical device (14) is configured to communicate with the second medical device (14) in response to the first medical device (14) being connected with the wireless network (16).

15. The system (10) of any one of claims 1 to 14, further comprising a control device (26), wherein the control device (26) and the medical device (14) are configured to electrically connect via a 1-Wire communication interface, wherein the control device (26) is configured to transmit a control signal and the medical device (14) is configured to receive the control signal (26) and perform a function based on the control signal.
